# EUROPEAN PATENT SPECIFICATION

(11) **EP 4 665 778 B1**
(45) Date of publication and mention of the grant of the patent: **09.12.2026**
(21) Application number: 24706238.3
(22) Date of filing: 14.02.2024
(51) Int. Cl.: C08G 18/24, C08G 18/38, C08G 18/48, C08G 18/66, C08G 18/73, C08G 18/75, C08G 83/00

(54) **PH-SWITCHABLE HYDROGEL**
PH-SCHALTBARES HYDROGEL
HYDROGEL COMMUTABLE EN PH

(30) Priority: 15.02.2023 NL 2034152
(43) Date of publication of application: 24.12.2025
(73) Proprietor: SupraPolix B.V., 5612 AX Eindhoven (NL)
(72) Inventor: MES, Tristan, 5612 AX Eindhoven (NL); BOSMAN, Anton Willem, 5612 AX Eindhoven (NL)
(74) Representative: Arnold & Siedsma
(86) International application number: PCT/NL2024/050075
(87) International publication number: WO 2024/172651

(56) References cited:
- EP-A1- 1 972 661
- WO-A1-2005/042641
- WO-A1-2016/018145
- WO-A1-2022/158978

## Description

### FIELD OF THE INVENTION

The present invention relates to a supramolecular polymer, to a process for the manufacture of a supramolecular polymer and to the supramolecular polymers that are obtained via said process. The present invention further relates to hydrogel formulations comprising said supramolecular polymer and to medical uses of said hydrogel formulations further comprising one or more pharmaceutically active compounds.

### BACKGROUND OF THE INVENTION

Hydrogels are three-dimensional networks of polymer chains with a high content of absorbed water molecules. Hydrogels find applications in for example medical applications, including bone transplants and tissue adhesives, barrier films, drug delivery systems, pharmaceutics, and in water management.

In the context of the present invention, a *'hydrogelformulation'* is a formulation that is either in the gelled state or is a liquid formulation that can be turned into a hydrogel upon application of an external stimulus such as pH or temperature and/or by chemical cross-linking. A *'hydrogel'* in the context of the present invention, on the other hand, always concerns the gelled stated of the hydrogel formulation.

Hydrogels can occur in the cross-linked form or in the uncross-linked form. Cross-linking of a hydrogel usually provides higher viscosities due to an apparent or real increase of the molecular weight. Cross-linking can be achieved chemically, *i.e.* by the formation of covalent bonds between different polymer chains, or physically by the formation of *e.g.* hydrogen bonds or ionic interactions between different polymer chains. Obviously, cross-linking can also be achieved by a combination of chemical and physical bonds.

Chemical cross-linking of hydrophilic polymers is a general and often applied route to obtain hydrogels. In order to be able to administer or process these gels, prepolymers are dissolved in water to provide an hydrogel formulation which is subsequently polymerized resulting in (*in situ*) hydrogel formation. Hydrogellation procedures are often based on the use of acrylic or methacrylic macromonomers that are not preferred in (biomedical) applications, because of their inherent toxicity and because they usually require an auxiliary, potentially hazardous, initiator for polymerization. Moreover, chemically cross-linked hydrogels lack reversibility and are limited in their degradation behaviour, as poly(acrylate)s and poly(methacrylate)s are not biodegradable. For example, US5410016A and J.A. Hubbell, J. Contr. Rel., 39, pp 305-313, 1996**,** disclose hydrogels based on copolymers of poly(ethylene glycol) with poly(DL-lactide) containing pendant acrylate functions that are cross-linked *in situ.* WO01/44307A2 discloses hydrogels based on polyvinyl alcohol modified with pendant acrylate and methacrylate groups that are chemically cross-linked *in situ.* According to these prior art references, an irreversible cross-linked hydrogel is obtained by starting from water processable prepolymers that contain reactive groups.

Hydrogels based on natural polymers, especially collagen, are biocompatible and mostly thermally reversible (see for example K.Y. Lee et al., Chem. Rev., 101, pp 1869-1879, 2001). However, the mechanical properties of these gels are limited and hardly, if at all, tunable. Especially the mechanical strength in these materials is too low, and often an additional chemical modification is required to make them stronger. However, this results in a reduced biocompatibility and a reduced biodegradation.

WO99/07343A1 discloses thermally reversible hydrogels intended for uses in drug delivery applications that are based on a hydrophilic polyethylene glycol block and hydrophobic PLLA (poly-L-lactic acid) blocks. The gelling is governed by the presence of the crystalline hard blocks formed by the PLLA. The presence of the crystalline PLLA-blocks limits the mechanical properties and the biodegradation of these materials to a great extent.

US5883211A discloses a thermo-reversible hydrogel comprising a physically cross-linked copolymer based on poly(acrylamide) containing up to six different monomers with hydrogen bonding *N*-substituent groups. The relative content of these monomers with hydrogen bonding *N*-substituent groups in the copolymer needs to be higher than 50% to display thermo-reversible gelling behaviour.

In general, *'supramolecular chemistry'* is understood to be the chemistry of physical or non-covalent, oriented, multiple (at least two), co-operative interactions. For instance, a *'supramolecular polymer'* is an organic compound that has polymeric properties - for example with respect to its rheological behaviour - due to specific and strong secondary interactions between the different molecules. These physical or non-covalent supramolecular interactions contribute substantially to the properties of the resulting material.

Supramolecular polymers comprising (macro)molecules that bear hydrogen bonding units can have polymer properties in bulk and in solution, because of the hydrogen bridges between the molecules. Sijbesma *et al.* (see US6320018B1 and Science, 278, pp 1601-1604, 1997) have shown that in cases where a self-complementary quadruple hydrogen bonding unit (4H-unit) is used, the physical interactions between the molecules become so strong that materials with much improved properties can be prepared.

EP1907482A discloses supramolecular hydrogel materials comprising water gellants that are comprised of hydrophilic polymers to which at least two 4H-units are covalently attached via urethane-alkyl moieties. The 4H-units can be present as end groups or in the polymer chain alternating with the hydrophilic polymer blocks. These hydrogels can be rendered liquid by increasing the temperature to at least 60 °C or by adding an organic water-miscible co-solvent. However, these reversible hydrogen-bonded hydrogel materials are insufficient in strength at low solids content and their viscosity is too high at biomedically relevant temperatures to allow administration via liquid processing techniques like injection through a syringe.

EP1972661A1 discloses supramolecular hydrogels that comprise isolated 4H-units linked via one or two urea bonding-motifs to a hydrophilic polymer. These hydrogels are thermo-reversible due to their supramolecular nature and the absence of covalent cross-links in the hydrogels. However, their reversible nature may also result in dissolving of the gel when an excess water is present, such as inside the body.

P.Y.W. Dankers et al., Adv. Mater., 24, 2012, pp 2703 -2709 disclose transient hydrogel networks based on specific embodiments of EP1972661A1, being supramolecular polymers consisting of polyethylene glycols which are end-functionalized with 4H-units via an urea-alkyl linker. They show that the formation of transient hydrogels is only possible for specific PEG-block lengths with specific alkyl spacer length. The authors ascribe their hydrogel properties to the formation of phase-separated stacks in the hydrogels composed of urea, alkyl and ureidopyrimidone (4H-unit) moieties. For example, a PEG 20 kDa block combined with a C₁₀-alkyl-urea spacer only results in a hydrogel when at least 15 wt.% polymer is present and when the temperature is equal or below 25 °C, whereas a PEG 10 kDa block combined with a C₁₀-alkyl-urea spacer only results in a hydrogel at temperatures below 40 °C when at least 10 wt.% polymer is dissolved. Moreover, the gels need to 'age' for at least 24 h to obtain these gel properties. Additionally, it is shown that these hydrogels fully dissolve within hours when excess of water is available. This, combined with their temperature sensitivity close to 37 °C and their strong concentration dependency, severely hampers their possible use in drug delivery applications, especially when long (weeks or longer) residence times are needed.

M.M.C. Bastings et al., Adv. Healthcare Mat., 3, 2014, pp 70-78 disclose that a hydrogel as described by Dankers 2012 (*vide supra*), consisting of a poly(ethyleneglycol) of 10 kDa blocks with only two 4H-unit end groups connected via an alkyl-urea-linker, can be rendered injectable when dissolved in strongly basic aqueous solution with a pH higher than 8.5. Again, a high solid content of 10 wt.% is needed to get hydrogel properties. Moreover, the elastic strength of the disclosed hydrogel is limited as displayed by the limited linear range that is lower than 100% deformation in strain sweep measurement and the fact that the hydrogel was easily crumbled manually at room temperature. The resulting hydrogel again needed several hours to build up its strength and was used for the release of growth factors in the myocardium.

US2017/210843A1, incorporated herein by reference in its entirety, discloses adhesive injectable hydrogels comprising isolated 4H-units and chemically cross-linkable moieties, such as dopamine, with adhesive properties towards tissue. These hydrogel polymers are liquids when dissolved at 15 wt.% in water and only form hydrogels after chemically cross-linking with an auxiliary chemical that is an oxidator. The hydrogels are disclosed to be useful in biomedical or cosmetic products for controlled drug delivery, tissue engineering, wound care, tissue-adhesion, or as tissue sealant, artificial cartilage material, cardiac patch, transdermal patch, cardio-vascular structure, and coating for a medical device. Likewise, WO2022/158978A1 discloses injectable hydrogel formulations comprising isolated 4H-units and chemically cross-linkable moieties, such as dopamine. These hydrogel polymers are liquids when dissolved at between 15 and 20 wt.% in water and only form hydrogels after chemically cross-linking with an auxiliary chemical. However, the need for a chemical (oxidative) reaction will limit the possibility to load the hydrogels with drugs that are chemically sensitive and might pose a risk for undesired reactions with surrounding tissues.

Because of the shortcomings of state-of-the-art hydrogels, there is a need for synthetic polymers that are able to gel water at low synthetic polymer concentrations and at a broad temperature range, without the need for chemical cross-linking. Furthermore, there is a need for hydrogels that have specific advantageous mechanical and elastic performances. Moreover, liquid hydrogel formulations comprising these synthetic polymers need to be injectable or otherwise easily processable in a liquid state, in order to facilitate easy processing and administration, implying that the hydrogels can be switched between a gelled state and a liquid state.

It is therefore an object of the invention to make biodegradable injectable hydrogel formulations that can be switched between a gelled state and a liquid state with advantageous mechanical and elastic performances in the gelled state.

It is a further object of the invention to provide synthetic polymers for use in those injectable hydrogel formulations.

### SUMMARY OF THE INVENTION

The inventors have unexpectedly found that one or more of the objects of the invention can be met by providing hydrogel formulations based on supramolecular polymers as defined herein. The inventors have unexpectedly established that the hydrogel formulations comprising the supramolecular polymer as defined herein behave liquid-like at a pH which is between 8.5 and 14.0 and at a temperature of between 20 and 40 °C. The corresponding dynamic viscosity at these conditions is low enough to inject the hydrogel formulations using for example a syringe equipped with a needle or a catheter. Moreover, they unexpectedly found that the hydrogel formulations comprising the supramolecular polymer as defined herein behave solid-like at a pH between 2.0 and less than 8.0 and at a temperature of 37 °C. At a pH between 2.0 and less than 8.0 and at a temperature of 37 °C, a gel is obtained with advantageous mechanical and elastic performances, even at low concentrations of the supramolecular polymer and without the need to chemically crosslink the polymer chains. Hence, the hydrogel formulations comprising the supramolecular polymer according to the invention can be switched between a liquid state and a gelled state using the pH of the hydrogel formulation and hydrogels are formed already at low concentrations of the supramolecular polymer and at a wide range of temperatures, thereby resulting in stable, yet injectable, hydrogels with favorable mechanical performances that are eminently suitable for applications such as drug delivery, barrier films, absorbents, anti-adhesion films and (dermal) fillers.

Accordingly, in a first aspect, the invention provides a supramolecular polymer comprising polymer chains according to Formula (I): 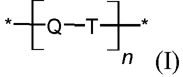 wherein:
- the average n in the supramolecular polymer is between 2 and 16, one of the building blocks Q is connected to a terminal group via the bond marked with an asterisk, and one of the building blocks T is connected to a terminal group via the bond marked with an asterisk;
- building block *-Q-* represents: 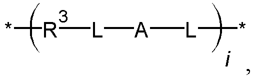 wherein the average i in the supramolecular polymer is between 1.5 and 6.0;
- building block *-T-* represents: 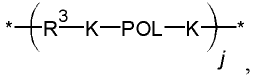 wherein the average j in the supramolecular polymer is between 1 and 6;
- the supramolecular polymer has an average molecular weight Mₙ of about 15 kDa to about 150 kDa, as determined with size-exclusion chromatography (SEC) with a GPC-system using RI detection with DMF comprising 10 mM LiBr at 50 °C as eluent, with the SEC-data being relative to PEO/PEG-standards;
- POL is a linear hydrophilic polymeric group having an average molecular weight Mₙ of about 1 kDa to about 30 kDa;
- moiety A represents moieties selected from the group consisting of Formulas (II-A) to (II-F), tautomers thereof and combinations thereof, wherein A is connected to L via the bonds marked with an asterisk in Formulas (II-A) to (II-F): 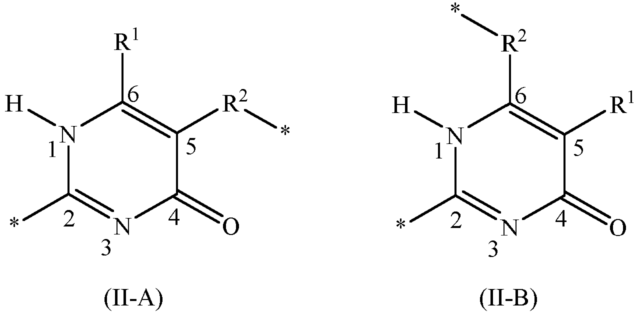 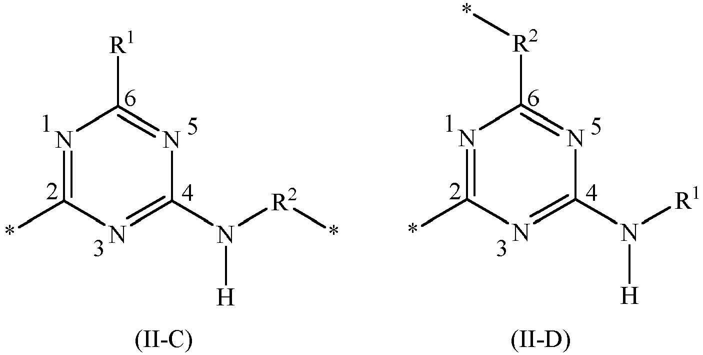 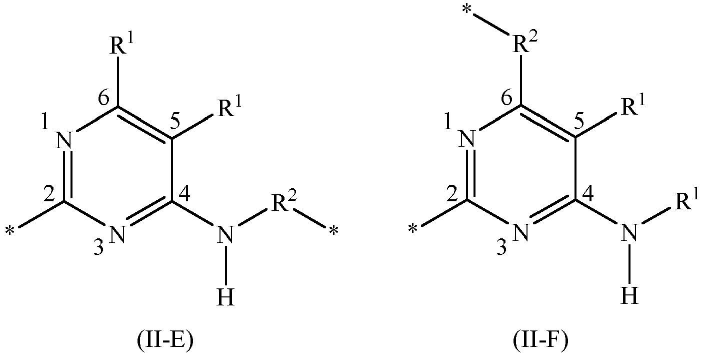
- each K is a urethane linking group;
- each L independently is a urethane or urea linking group, with the proviso that any moiety A according to Formula (II-A) and (II-B) is always coupled to a urea linking group L on the 2-position of the 4-pyrimidone, any moiety A according to Formula (II-C) and (II-D) is always coupled to a urea linking group L on the 2-position of the triazine, and any moiety A according to Formula (II-E) and (II-F) is always coupled to a urea linking group L on the 2-position of the pyrimidine;
- each R¹ is independently selected from the group consisting of hydrogen and C₁ - C₂₀ alkyl;
- R² is selected from the group consisting of C₁ - C₂₀ alkylene, optionally substituted with O or S; and
- R³ is selected from the group consisting of linear or branched C₂ - C₂₀ alkylene groups and cyclic C₃ - C₂₄ alkylene groups.

In a second aspect, the invention provides a process for the manufacture of a supramolecular polymer, preferably a supramolecular polymer according to the first aspect, by reacting, optionally in a non-reactive solvent, a compound A' selected from the group consisting of Formulas (III-A) to (III-F), tautomers thereof and combinations thereof with a diisocyanate compound C' according to the Formula O=C=N-R³-N=C=O and a polymer HO-POL-OH: 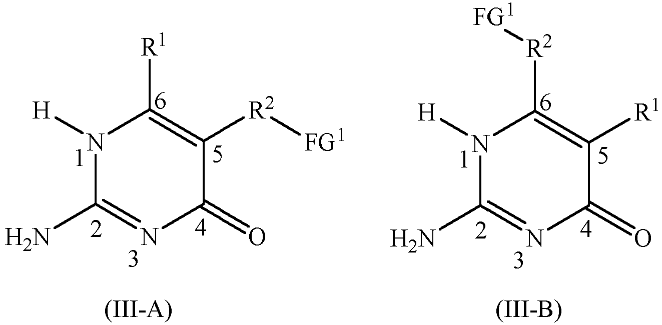 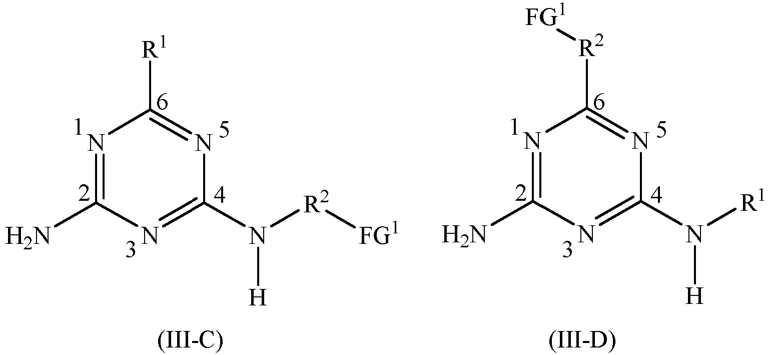 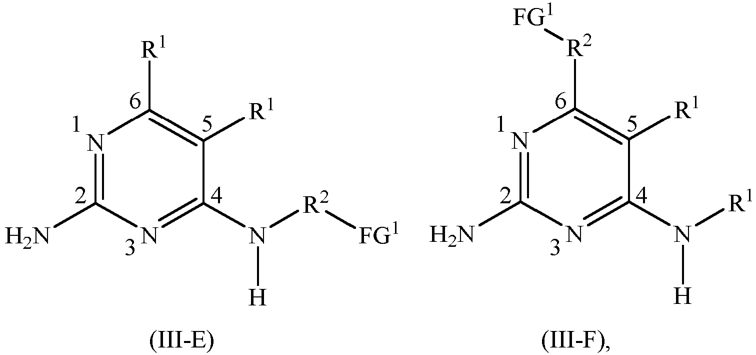
wherein R¹, R², R³ and POL are as defined in the context of the first aspect,
wherein FG¹ represents a functional group selected from OH and NH₂,
wherein the molar ratio of compound A' to HO-POL-OH applied during the reaction is between 1.5 : 1.0 and 6.0 : 1.0, and
wherein the molar ratio of compound C' to the sum of compound A' and HO-POL-OH applied during the reaction is between 1.1 : 1.0 and 0.9 : 1.0.

In a third aspect, the invention provides a supramolecular polymer obtained by or obtainable by the process according to the second aspect.

In a fourth aspect, the invention provides a hydrogel formulation comprising 50.0 - 99.7 wt.% of water, 0.3 - 50.0 wt.% of the supramolecular polymer according to the first aspect or the third aspect, and 0 - 30 wt.% of further ingredients, based on the weight of the hydrogel formulation, wherein the amounts of water, supramolecular polymer and further ingredients add up to 100 wt.% of the hydrogel formulation.

In a fifth aspect, the invention provides a hydrogel formulation according to the fourth aspect having a pH between 8.5 and 14.0, which is a liquid at a temperature of between 20 and 40 °C, and comprising 0.0001 to 30 wt.% of one or more pharmaceutically active ingredients, based on the weight of the hydrogel formulation, for use in the treatment of oncological diseases, cardio-vascular diseases, orthopaedic diseases, gastrointestinal diseases or wound care in mammalian subjects, said treatment comprising injecting the hydrogel formulation into the mammalian body, followed by release of the one or more pharmaceutically active ingredients from the hydrogel formulation.

In a sixth aspect, the invention provides a hydrogel formulation according to the fourth aspect having a pH between 8.5 and 14.0, which is a liquid at a temperature of between 20 and 40 °C, and comprising 0.0001 to 30 wt.% of one or more pharmaceutically active ingredients, based on the weight of the hydrogel formulation, for use in a method of prevention of tissue adhesion or in reconstructive surgery or cosmetic surgery in mammalian subjects, said method comprising injecting the hydrogel formulation into the mammalian body, followed by release of the one or more pharmaceutically active ingredients from the hydrogel formulation.

In a seventh aspect, the invention concerns a hydrogel formulation according to the fourth aspect having a pH between 2.0 and less than 8.0, which is a gel at a temperature of between 20 and 40 °C, and comprising 0.0001 to 30 wt.% of one or more pharmaceutically active ingredients, based on the weight of the hydrogel formulation, for use in a method of treatment or prevention of bacterial or viral infections in a mammal, said method comprising applying the hydrogel formulation onto the mammalian body, preferably onto the skin of the mammalian body.

### GENERAL DEFINITIONS

The *'hydroxyl value'* is defined as the number of milligrams of potassium hydroxide required to neutralize the acetic acid taken up on acetylation of one gram of a chemical substance that contains free hydroxyl groups. Hydroxyl value is a measure of the content of free hydroxyl groups in a chemical substance, usually expressed in units of the mass of potassium hydroxide (KOH) in milligrams equivalent to the hydroxyl content of one gram of the chemical substance.

The term *'obtainable by'* is considered to be synonymous to *'obtained by'.*

The term *'one step reaction'* as used herein refers to a *'one pot reaction',* wherein all reactants are present at the same time and are added substantially simultaneously, as opposed to a reaction comprising *'sequential reaction steps'* wherein a subsequent reactant is added after (at least partial) completion of a previous reaction step, possibly in different reaction vessels.

### DETAILED DESCRIPTION

### Supramolecular polymer

In a first aspect, the invention concerns a supramolecular polymer comprising polymer chains according to Formula (I): 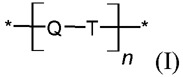 wherein:
- the average n in the supramolecular polymer is between 2 and 16, one of the building blocks Q is connected to a terminal group via the bond marked with an asterisk, and one of the building blocks T is connected to a terminal group via the bond marked with an asterisk;
- building block *-Q-* represents: 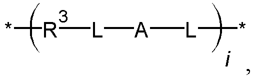 wherein the average i in the supramolecular polymer is between 1.5 and 6.0;
- building block *-T-* represents: 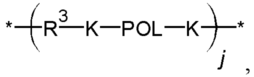 wherein the average j in the supramolecular polymer is between 1 and 6;
- the supramolecular polymer has an average molecular weight Mₙ of about 15 kDa to about 150 kDa, as determined with size-exclusion chromatography (SEC) with a GPC-system using RI detection with DMF comprising 10 mM LiBr at 50 °C as eluent, with the SEC-data being relative to PEO/PEG-standards;
- POL is a linear hydrophilic polymeric group having an average molecular weight Mₙ of about 1 kDa to about 30 kDa;
- moiety A represents moieties selected from the group consisting of Formulas (II-A) to (II-F), tautomers thereof and combinations thereof, wherein A is connected to L via the bonds marked with an asterisk in Formulas (II-A) to (II-F): 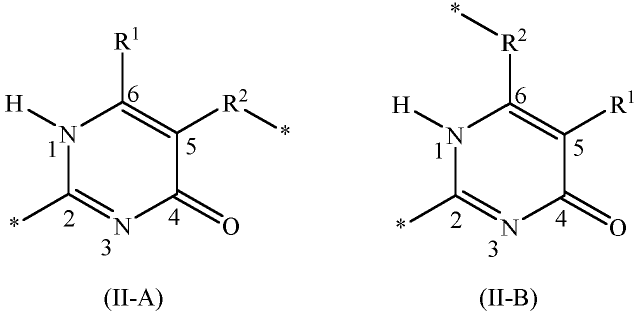 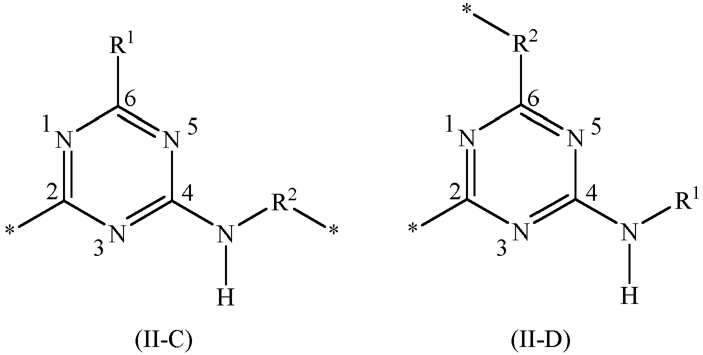 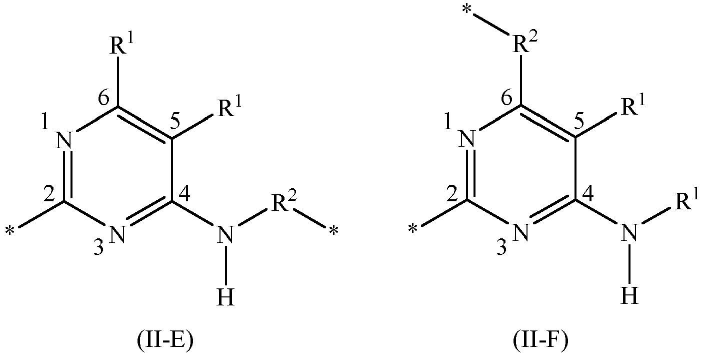
- each K is a urethane linking group;
- each L independently is a urethane or urea linking group, with the proviso that any moiety A according to Formula (II-A) and (II-B) is always coupled to a urea linking group L on the 2-position of the 4-pyrimidone, any moiety A according to Formula (II-C) and (II-D) is always coupled to a urea linking group L on the 2-position of the triazine, and any moiety A according to Formula (II-E) and (II-F) is always coupled to a urea linking group L on the 2-position of the pyrimidine;
- each R¹ is independently selected from the group consisting of hydrogen and C₁ - C₂₀ alkyl;
- R² is selected from the group consisting of C₁ - C₂₀ alkylene, optionally substituted with O or S; and
- R³ is selected from the group consisting of linear or branched C₂ - C₂₀ alkylene groups and cyclic C₃ - C₂₄ alkylene groups.

The terms *'POL'* and '**-POL-*'* in the context of the first aspect are used interchangeably and both concern a linear hydrophilic polymeric group that is connected to other groups, such as via the bonds indicated with an asterisk. Likewise, the terms *'R²'* and '*-*R²*-*', the terms '*R³*' and '*-*R³*-*', the terms *'K'* and '*-*K*-*', the terms *'L'* and '**-L-*'* and the terms *'A'* and `*-*A*-*' are used interchangeably.

As will be appreciated by those skilled in the art, the term *'supramolecular polymer'* as used herein does not concern a single polymer chain, but is the result of a statistical copolymerization process and therefore concerns a composition comprising copolymer chains of varying chain composition and varying chain length. The individual polymer chains each comprise one or more *-Q-T-* repeating units, wherein each block *-Q-* in any one of the one or more repeating units *-Q-T-* can individually consist of one or more repeating units according to *-R³-L-A-L-*. Likewise, the individual polymer chains each comprise one or more *-Q-T-* repeating units, wherein each block *-T-* in any one of the one or more repeating units *-Q-T-* can individually consist of one or more repeating units according to *- R³-K-POL-K-*.

Due to the stoichiometry of the reactants used during the reaction to produce the supramolecular polymer (see in this respect the process according to the second aspect), more in particular the molar ratio of the bifunctional monomer resulting in group *-A-* to the bifunctional macromonomer resulting in group *-POL-* in the supramolecular polymer being equal to or higher than 1.5 : 1.0, the average number of repeating units i according to *-R³-L-A-L-* across the different repeating units *- Q- T-* in all the copolymer chains constituting the supramolecular polymer is equal to or higher than 1.5. Accordingly, the supramolecular polymer comprises a relatively large number of polymer chains having blocks *-Q-* such as *-R³-L-A-L-R³-L-A-L-*, *-R³-L-A-L-R³-L-A-L-R³-L-A-L-*, etc.

In a preferred embodiment, the average n in the supramolecular polymer is between 3 and 12, such as between 4 and 10 or between 5 and 9.

In another preferred embodiment, the average n in the supramolecular polymer is between 2 and 15, such as between 2 and 13, between 2 and 12, between 2 and 10, between 2 and 8 or between 2 and 6.

In yet another preferred embodiment, the average n in the supramolecular polymer is between 3 and 16, such as between 4 and 16, between 5 and 16, between 6 and 16, between 7 and 16 or between 8 and 16.

In a preferred embodiment, the average i in the supramolecular polymer is between 2 and 5, such as between 3 and 4.

In another preferred embodiment, the average i in the supramolecular polymer is between 1.5 and 5.5, such as between 1.5 and 5.0, between 1.5 and 4.5, between 1.5 and 4.0, between 1.5 and 3.5 or between 1.5 and 3.0.

In yet another preferred embodiment, the average i in the supramolecular polymer is between 1.8 and 6.0, such as between 2.0 and 6.0, between 2.2 and 6.0, between 2.4 and 6.0, between 2.6 and 6.0 or between 2.8 and 6.0.

In a preferred embodiment, the average j in the supramolecular polymer is between 1.5 and 5, such as between 2 and 3.

In another preferred embodiment, the average j in the supramolecular polymer is between 1 and 5, such as between 1 and 4, between 1 and 3, between 1 and 2, between 1 and 1.5 or between 1 and 1.1.

In yet another preferred embodiment, the average j in the supramolecular polymer is between 1.1 and 6, such as between 1.2 and 6, between 1.5 and 6, between 2 and 6, between 2.5 and 6 or between 3 and 6.

As defined hereinbefore, the supramolecular polymer according to Formula (I) has an average molecular weight Mₙ of about 15 kDa to about 150 kDa, as determined with size-exclusion chromatography (SEC) with a GPC-system using RI detection with DMF comprising 10 mM LiBr at 50 °C as eluent, with the SEC-data being relative to PEO/PEG-standards.

In a preferred embodiment, the supramolecular polymer according to Formula (I) has an average molecular weight Mₙ between 20 kDa and 140 KDa, more preferably between 30 kDa and 130 kDa, such as between 40 kDa and 120 kDa or between 50 kDa and 110 kDa.

In another preferred embodiment, the supramolecular polymer according to Formula (I) has an average molecular weight Mₙ between 20 kDa and 150 KDa, such as between 30 kDa and 150 kDa, between 40 kDa and 150 kDa, between 50 kDa and 150 kDa, between 60 kDa and 150 kDa or between 70 kDa and 150 kDa.

In yet another preferred embodiment, the supramolecular polymer according to Formula (I) has an average molecular weight Mₙ between 15 kDa and 145 KDa, such as between 15 kDa and 130 kDa, between 15 kDa and 120 kDa, between 15 kDa and 110 kDa or between 15 kDa and 100 kDa.

### Group *-POL-*

The linear hydrophilic polymeric groups *-POL-* in the supramolecular polymer may comprise any type of hydrophilic polymer backbone known in the art.

The linear hydrophilic polymeric groups *-POL-* in the supramolecular polymer have a number average molecular weight (Mₙ) that is preferably determined by end group titration of the telechelic macromonomer of which the linear hydrophilic polymeric groups *-POL-* are formed, such as hydroxy value determination or standard test method ASTM E222-10.

As indicated hereinbefore, the linear hydrophilic polymeric groups *-POL-* in the supramolecular polymer have an average molecular weight Mₙ of about 1 kDa to about 30 kDa.

In a preferred embodiment, the linear hydrophilic polymeric groups *-POL-* in the supramolecular polymer have an average molecular weight Mₙ between 8 kDa and 28 kDa, more preferably between 10 kDa and 25 kDa, such as between 12 kDa and 23 kDa or between 14 kDa and 21 kDa.

In another preferred embodiment, the linear hydrophilic polymeric groups *-POL-* in the supramolecular polymer have an average molecular weight Mₙ between 9 kDa and 30 kDa, such as between 10 kDa and 30 kDa, between 12 kDa and 30 kDa, between 14 kDa and 30 kDa, between 16 kDa and 30 kDa or between 18 kDa and 30 kDa.

In yet another preferred embodiment, the linear hydrophilic polymeric groups *-POL- * in the supramolecular polymer have an average molecular weight Mₙ between 8 kDa and 29 KDa, such as between 8 kDa and 29 kDa, between 8 kDa and 26 kDa, between 8 kDa and 24 kDa or between 8 kDa and 22 kDa.

In a preferred embodiment, the linear hydrophilic polymeric groups *-POL-* in the supramolecular polymer have an average molecular weight Mₙ between 1 kDa and 20 kDa, such as between 2 kDa and 12 kDa, between 3 kDa and 8 kDa or between 4 kDa and 6 kDa.

In another preferred embodiment, the linear hydrophilic polymeric groups *-POL- * in the supramolecular polymer have an average molecular weight Mₙ between 1 kDa and 18 kDa, such as between 1 kDa and 16 kDa, between 1 kDa and 14 kDa, between 1 kDa and 12 kDa, between 1 and 10 kDa or between 1 kDa and 8 kDa.

In yet another preferred embodiment, the linear hydrophilic polymeric groups *-POL- * in the supramolecular polymer have an average molecular weight Mₙ between 2 kDa and 20 kDa, such as between 3 kDa and 20 kDa, between 4 kDa and 20 kDa or between 5 kDa and 20 kDa.

The linear hydrophilic polymeric groups *-POL-* in the supramolecular polymer can be of natural origin or of synthetic origin. Preferably, the linear hydrophilic polymeric groups *-POL-* are of synthetic origin. If the linear hydrophilic polymeric groups *-POL-* are hydrophilic and of natural origin, they are preferably selected from the group consisting of proteins (e.g. proteins selected from the group consisting of collagen, gelatin, and fibrin), polysaccharides (e.g. polysaccharides selected from the group consisting of hyaluronic acid, dextran, agar, agarose, xantham gums, natural gum, alginate, chitosan and inulin) and synthetic derivatives of these polymers of natural origin, preferably gelatin or chitosan.

If the linear hydrophilic polymeric groups *-POL-* are of synthetic origin, they can be any synthetic linear polymeric group, preferably having a solubility in water of at least 1 g/L at 20 °C, more preferably at least 10 g/L at 20 °C.

The linear hydrophilic polymeric group *-POL-* is preferably selected from the group consisting of polyethers, polyesters, polycarbonates, polyamides, polyoxazolines, polyacrylates, polymethacrylates, polyolefins, hydrogenated polyolefins, polysiloxanes, polycarbonates, (per)fluorinated polyethers, polyvinylenes, or co-polymers of such polymers, and combinations thereof. More preferably, the linear hydrophilic polymeric group *-POL-* is selected from the group consisting of polyethers, polyesters, polycarbonates, polyamides, polyoxazolines, polyacrylates, polymethacrylates, co-polymers of such polymers and combinations thereof. Even more preferred are polyethers, polyesters, polycarbonates, polyoxazolines or copolymers thereof. Although some of the above listed linear polymeric groups *-POL-* themselves may not be hydrophilic per se, co-polymerizing them with the right amount of water-soluble linear hydrophilic polymeric groups *-POL-*, or use of a combination of these linear polymeric groups *-POL- *, may lead to hydrophilic character.

In a very preferred embodiment, the linear hydrophilic polymeric group *-POL-* is selected from polyethylene glycols, most preferably a linear polyethylene glycol having an average molecular weight Mₙ of between 10 kDa and 25 kDa.

In another very preferred embodiment, the linear hydrophilic polymeric group *-POL-* is a linear polyethylene glycol having an average molecular weight Mₙ of between 3 kDa and 8 kDa

### Group *-A-* and *-L-A-L-*

As defined hereinbefore, each R¹ in moiety *-A-* is independently selected from the group consisting of hydrogen and C₁ - C₂₀ alkyl. The C₁ - C₂₀ alkyl group can be cyclic (for C₃ - C₂₀ alkyl), branched (for C₃ - C₂₀ alkyl), or linear, preferably linear. In a preferred embodiment, each R¹ is independently selected from a C₁ - C₁₃ alkyl group. More preferably, each R¹ is independently selected from the group consisting of methyl, ethyl, propyl, isopropyl, n-butyl, t-butyl, n-hexyl, cyclohexyl, 3-ethylpentyl and tredecyl. Most preferably, R¹ is methyl.

In a preferred embodiment:
- any moiety *-A-* according to Formula (II-A) and (II-B) is coupled to a urea linking group L on the 2-position of the 4-pyrimidone and to a urethane urea linking group L on the 5-position of the 4-pyrimidone;
- any moiety *-A-* according to Formula (II-C) and (II-D) is coupled to a urea linking group L on the 2-position of the triazine and to a urethane linking group L on the 4-position of the triazine,
- any moiety *-A-* according to Formula (II-E) and (II-F) is coupled to a urea linking group L on the 2-position of the pyrimidine and to a urethane linking group L on the 4-position of the pyrimidine.

In a preferred embodiment, *-A-* in the supramolecular polymer according to Formula (I) is selected from the group consisting of Formula (II-A).

In a very preferred embodiment, *-A-* in Formula (I) represents the following moieties selected from the group consisting of Formula (II-A): 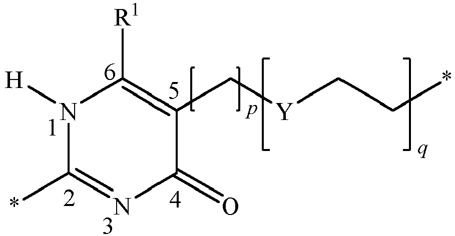 wherein:
R¹ is selected from the group consisting of hydrogen and C₁ - C₂₀ alkyl;
Y is O or S;
*p* is an integer of 1 to 20; and
*q* is an integer of 0 to 8.

In even more preferred embodiments, *-A-* in Formula (I) represents the following moieties selected from the group consisting of Formula (II-A): 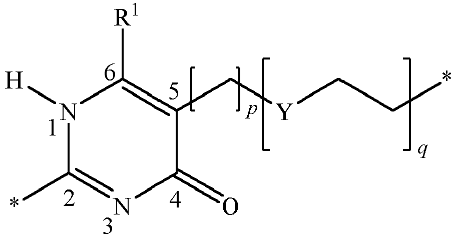 wherein R¹ is methyl and:
a) *p* is 2 and *q* is 0;
b) *p* is 2, *q* is 1 and Y is O; or
c) *p* is 4 to 11 and *q* is 0.

In a most preferred embodiment, *-A-* in Formula (I) represents:

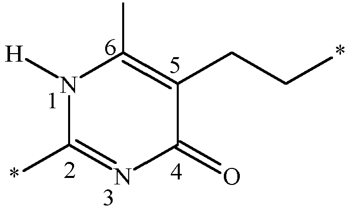

In a very preferred embodiment, *-L-A-L-* in Formula (I) represents the following moieties: 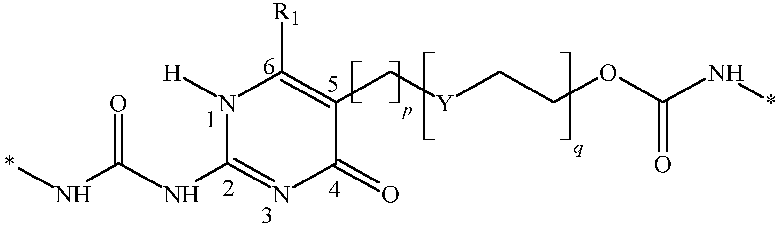 wherein:
R¹ is selected from the group consisting of hydrogen and C₁ - C₂₀ alkyl;
Y is O or S;
*p* is an integer of 1 to 20; and
*q* is an integer of 0 to 8.

In even more preferred embodiments, *-L-A-L-* in Formula (I) represents the following moieties selected from the group consisting of Formula (II-A): 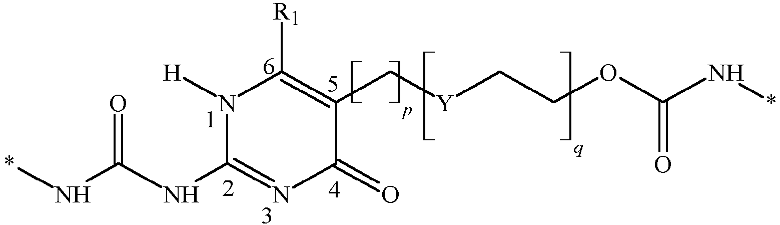 wherein R¹ is methyl and:
a) *p* is 2 and *q* is 0;
b) *p* is 2, *q* is 1 and Y is O; or
c) *p* is 4 to 11 and *q* is 0.

In a most preferred embodiment, *-L-A-L-* in Formula (I) represents:

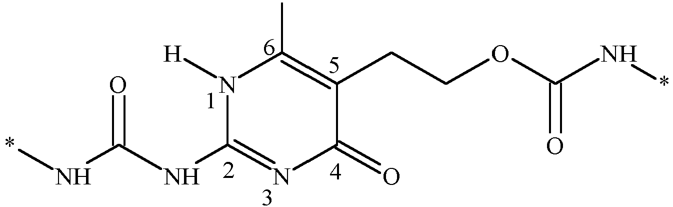

### Group *-R³-*

As defined hereinbefore, *-R³-* is selected from the group consisting of linear or branched C₂ - C₂₀ alkylene groups and cyclic C₃ - C₂₄ alkylene groups. In a preferred embodiment, *- R³- * is selected from the group consisting of linear C₂ - C₂₀ alkylene groups, such as butylene, hexylene and dodecylene. In another preferred embodiment, *-R³-* is selected from hexylene, 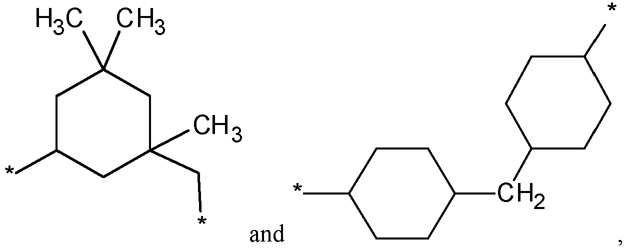 preferably from

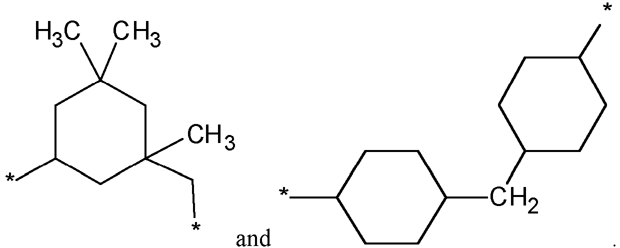

Most preferably, *- R³- * is:

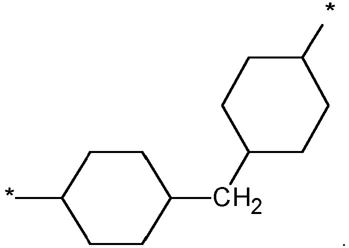

### Terminal groups

As defined hereinbefore, one of the blocks Q in the supramolecular polymer according to Formula (I) is connected to a terminal group via the bond marked with an asterisk, and one of the blocks T in the supramolecular polymer according to Formula (I) is connected to a terminal group via the bond marked with an asterisk. The nature of these terminal groups is not particularly relevant to the invention.

As will be appreciated by those skilled in the art, the average molecular weight Mₙ of the supramolecular polymer according to Formula (I) as defined hereinbefore also includes both terminal groups.

The supramolecular polymer according to Formula (I) with explicit terminal groups E and F can be depicted with Formula (Ia):

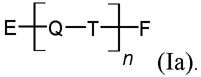

As will be appreciated by those skilled in the art, the nature of these terminal groups is dictated by the process used to manufacture the supramolecular polymer according to Formula (I). See in this respect for example the process according to the second aspect.

In a preferred embodiment, terminal group E is chosen from the group consisting of:
(a) *-N=C=O;
(b) optionally *-NH₂;
(c) *-T-N=C=O;
(d) optionally *-T-NH₂;
(e) *-T-L-A-NH₂, wherein the NH₂ group is connected via a bond to the 2-position or via a bond to the group R² of the structure according to any one of Formulas (II-A) to (II-F);
(f) *-T-L-A-OH, wherein the OH group is connected via a bond to the group R² of the structure according to any one of Formulas (II-A) to (II-F);
(g) *-T-K-POL-OH;
(h) *-K-POL-OH;
(i) *-L-A-NH₂, wherein the NH₂ group is connected via a bond to the 2-position or via a bond to the group R² of the structure according to any one of Formulas (II-A) to (II-F);
(j) *-L-A-OH, wherein the OH group is connected via a bond to the group R² of the structure according to any one of Formulas (II-A) to (II-F);
(k) a combination of (a) to (j).

In a preferred embodiment, terminal group F is chosen from the group consisting of:
(l) *-Q-R³-N=C=O;
(m) optionally *-Q-R³-NH₂;
(n) *-Q-R³-K-POL-OH;
(o) *-Q-R³-L-A-NH₂, wherein the NH₂ group is connected via a bond to the 2-position or via a bond to the group R² of the structure according to any one of Formulas (II-A) to (II-F);
(p) *-Q-R³-L-A-OH, wherein the OH group is connected via a bond to the group R² of the structure according to any one of Formulas (II-A) to (II-F);
(q) *-R³-N=C=O;
(r) optionally *-R³-NH₂;
(s) *-R³-K-POL-OH;
(t) *-R³-L-A-NH₂, wherein the NH₂ group is connected via a bond to the 2-position or via a bond to the group R² of the structure according to any one of Formulas (II-A) to (II-F);
(u) *-R³-L-A-OH, wherein the OH group is connected via a bond to the group R² of the structure according to any one of Formulas (II-A) to (II-F);
(v) a combination of (l) to (u).

In a very preferred embodiment, the terminal group E is chosen from the group consisting of (a) - (j) defined hereinbefore, preferably a combination of (a) to (j), and the terminal group F is chosen from the group consisting of (l) - (u) defined hereinbefore, preferably a combination of (l) to (u).

### Process for the manufacture of a supramolecular polymer

In a second aspect, the invention concerns a process for the manufacture of a supramolecular polymer, preferably a supramolecular polymer according to the first aspect, by reacting, optionally in a non-reactive solvent, a compound A' selected from the group consisting of Formulas (III-A) to (III-F), tautomers thereof and combinations thereof with a diisocyanate compound C' according to the Formula O=C=N-R³-N=C=O and a polymer HO-POL-OH: 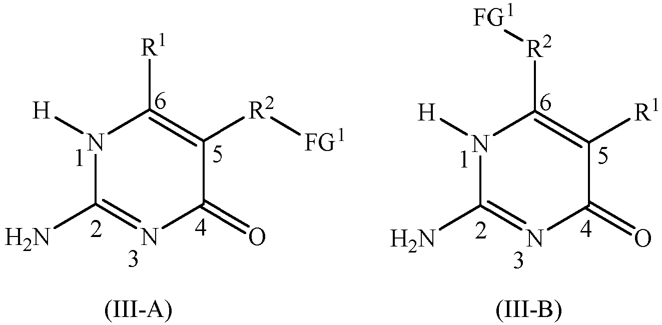 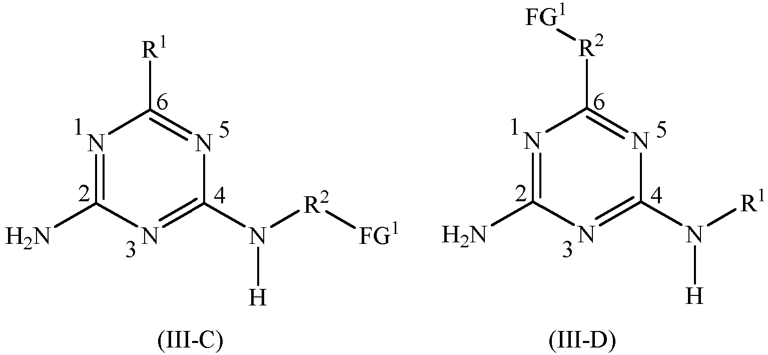 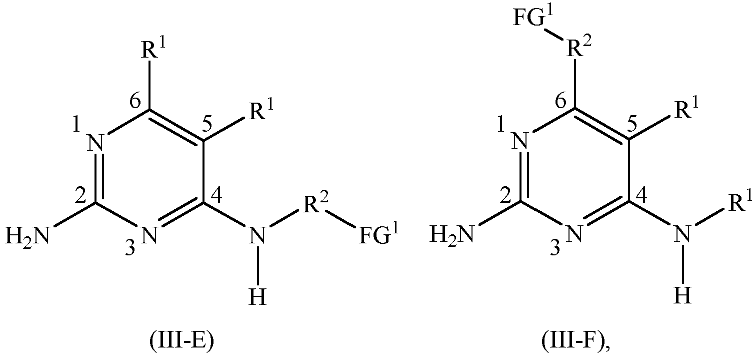
wherein R¹, R², R³ and POL are as defined in the context of the first aspect,
wherein FG¹ represents a functional group selected from OH and NH₂, preferably OH,
wherein the molar ratio of compound A' to HO-POL-OH applied during the reaction is between 1.5 : 1.0 and 6.0 : 1.0, and
wherein the molar ratio of compound C' to the sum of compound A' and HO-POL-OH applied during the reaction is between 1.1 : 1.0 and 0.9 : 1.0.

In a preferred embodiment, the molar ratio of compound A' to HO-POL-OH applied during the reaction is between 2 : 1.0 and 6.0 : 1.0, such as between 2.5 : 1.0 and 6.0 : 1.0 or between 3.0 : 1.0 and 6.0 : 1.0, and wherein the molar ratio of compound C' to the sum of compound A' and HO-POL-OH applied during the reaction is between 1.1 : 1.0 and 0.9 : 1.0.

In another preferred embodiment, the molar ratio of compound A' to HO-POL-OH applied during the reaction is between 1.5 : 1.0 and 5.5 : 1.0, such as between 1.5 : 1.0 and 5.0 : 1.0, between 1.5 : 1.0 and 4.5 : 1.0 or between 1.5 : 1.0 and 4.0 : 1.0, and wherein the molar ratio of compound C' to the sum of compound A' and HO-POL-OH applied during the reaction is between 1.1 : 1.0 and 0.9 : 1.0.

Due to the stoichiometry of the reactants used during the reaction to produce the supramolecular polymer, more in particular the molar ratio of the compound A' to the polymer HO-POL-OH being equal to or higher than 1.5 : 1:0, blocks of the following repeating units *-[R³-L-A-L]-* are formed, such as for example *-R³-L-A-L-R³-L-A-L-*, *-R³-L-A-L-R³-L-A-L-R³-L-A-L-*, etc.

The process for the manufacture of the supramolecular polymer can be performed by any method known in the art, for example in solution, in the bulk or using reactive extrusion. The process is, irrespective of whether it is performed as a one step process or as a sequential process comprising two or more reaction steps, preferably performed at a temperature between about 20 °C and about 140 °C, more preferably between about 60 °C and about 120 °C, and most preferably between about 80 °C and about 100 °C.

In a very preferred embodiment, the process is performed as a one step reaction or one pot reaction.

The process for the preparation of the supramolecular polymer may be performed in the presence of a catalyst. Examples of suitable catalysts promoting the reaction between isocyanates and hydroxyl groups are known in the art. Preferred catalysts include tertiary amines and catalysts comprising a metal. Preferred tertiary amines are 1,4-diazabicyclo[2.2.2]octane (DABCO) and 1,8-diazabicyclo[5.4.0]undec-7-ene (DBU). Preferred catalysts comprising a metal are tin(IV) compounds and zirconium(IV) compounds, preferably selected from the group consisting of tin(II)octanoate, dibutyltin(IV)laurate and zirconium(IV)acetoacetate. Most preferably, the catalyst is dibutyltin(IV)laurate, tin(II)octanoate or zirconium(IV)acetoacetate. The amount of catalyst is generally below about 1% by weight, preferably below about 0.2% by weight and most preferably in between 0.005 and 0.05% by weight, based on the total amount of the reactants.

In an embodiment, the process is performed in the presence of a non-reactive solvent, preferably a non-reactive aprotic organic solvent. It is also preferred that the reaction mixture does not comprise any inorganic solvents such as water. Non-reactive aprotic organic solvents are preferably selected from ethers, such as diethyl ether, THF, methyl-tetrahydrofuran, dioxane and methyl-tert-butyl ether, DMSO, dimethyl acetamide, DMF, NMP, trialkyl amines, chloroform, dichloromethane, diethylcarbonate, propylene carbonate, ketones such as acetone, MEK and methyl-tert-butyl ketone, esters such as ethyl acetate, 2-methoxy-ethyl-acetate and butyl acetate, and toluene. Most preferably, the non-reactive aprotic organic solvent is dimethylformamide.

Preferably, the non-reactive solvent is present in an amount below about 50% by weight, more preferably below about 20% by weight, even more preferably below about 10% by weight and most preferably in between 5 and 1% by weight, based on the total amount of the reactants.

In certain embodiments, a solvent can be dispensed with, such as in reactive extrusion or in a one pot reaction wherein HO-POL-OH acts as a reactive solvent for the remaining reactants.

The supramolecular polymer can be isolated as such, *i.e.* as polymer in solvent, or can be isolated as a powder after precipitation in a non-solvent, chopped into pellets, spun into fibers, extruded into films, directly dissolved in a medium of choice, or transformed or formulated into whatever form that is desired. Most preferably, the supramolecular polymer is isolated in alkanol/water mixtures or water at pH > 8.5.

### HO-POL-OH

The group *-POL-* is as defined in the context of the first aspect The polymer (macromonomer) HO-POL-OH preferably is bifunctional (telechelic), but small deviations from this bifunctionality are also encompassed by the invention.

In preferred embodiments, the polymer according to Formula HO-POL-OH has about 1.8 to about 2 end-groups OH, preferably about 1.9 to about 2 end groups OH, most preferably higher than about 1.95 to about 2 end groups OH.

### Compound A '

In a preferred embodiment, compound A' is selected from the group consisting of Formula (III-A).

In a very preferred embodiment, A' represents the following compounds selected from the group consisting of Formula (III-A): 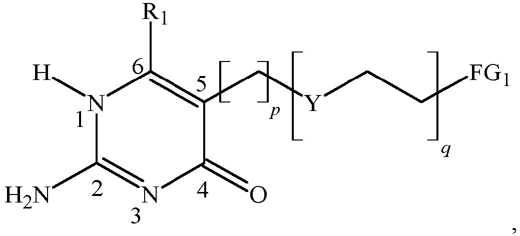 wherein:
R¹ is selected from the group consisting of hydrogen and C₁ - C₂₀ alkyl;
Y is O or S;
*p* is an integer of 1 to 20; and
*q* is an integer of 0 to 8.

In even more preferred embodiments, compound A' represents the following moieties selected from the group consisting of Formula (II-A): 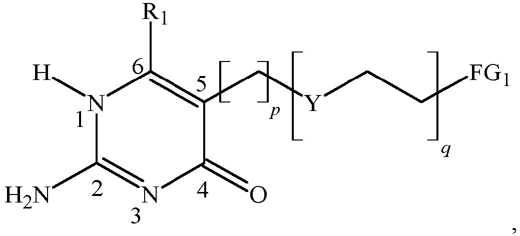 wherein R¹ is methyl and:
a) *p* is 2, *q is* 0 and FG¹ is OH;
b) *p* is 2, *q is* 1, Y is O and FG¹ is OH;
c) *p* is 4 to 11, *q* is 0 and FG¹ is OH; or
d) *p* is 4 to 11, *q* is 0 and FG¹ is NH₂.

In a most preferred embodiment, compound A' represents:

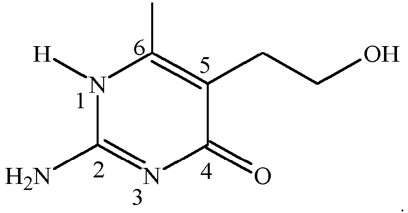

### Compound C'

In a preferred embodiment, compound C' is selected from 1,6-diisocyanatohexane, isophorone diisocyanate and 4,4'-methylene-bis(cyclohexyl isocyanate), more preferably from isophorone diisocyanate and 4,4'-methylene-bis(cyclohexyl isocyanate) and most preferably 4,4'-methylene-bis(cyclohexyl isocyanate).

### Supramolecular polymer obtainable by the process

In a third aspect, the invention concerns a supramolecular polymer obtained by or obtainable by the process according to the second aspect.

### Hydrogel formulation

In a fourth aspect, the invention concerns a hydrogel formulation comprising 50.0 - 99.7 wt.% of water, 0.3 - 50.0 wt.% of the supramolecular polymer according to the first aspect or the third aspect, and 0 - 30 wt.% of further ingredients, based on the weight of the hydrogel formulation, wherein the amounts of water, supramolecular polymer and further ingredients add up to 100 wt.% of the hydrogel formulation.

The hydrogel formulation preferably comprises 0.5 - 30 wt.% of the supramolecular polymer according to the first aspect or the third aspect, more preferably 0.8 - 20 wt.%, even more preferably 0.9 - 10 wt.%, even more preferably 0.95 - 8 wt.%, and most preferably 1.0 - 5 wt.%, based on the weight of the hydrogel formulation.

The hydrogel formulation preferably comprises 70 - 95 wt.% of water, more preferably 75 - 90 wt.%, most preferably 78 - 87 wt.%, based on the weight of the hydrogel formulation.

The hydrogel formulation preferably comprises 0.0001 - 25 wt.% of further ingredients, more preferably 0.001 - 20 wt.%, even more preferably 0.01 - 15 wt.%, most preferably 0.025 - 5 wt.%, based on the weight of the hydrogel formulation.

In another embodiment, the hydrogel formulation comprises 0.0001 to 20 wt.% of further ingredients, preferably 0.01 to 10 wt.%, and most preferably 0.1 to 5 wt.%, based on the weight of the hydrogel formulation.

The optional further ingredients are functional ingredients that will contribute to the specific use of the hydrogel formulation. Preferably, the hydrogel formulation contains a base to adjust the pH. The base can be any base, organic or inorganic, and is preferably an inorganic base, in which the inorganic base is preferably chosen from the group consisting of alkali hydroxides, such as NaOH, alkali phosphates, alkali hydrogenphosphates, alkali dihydrogenphosphates, alkali pyrophosphates, alkali hydrogenpyrophosphates and combinations thereof.

In another preferred embodiment, the hydrogel formulation comprises as a further ingredient an additional polymer in order to modify the rheological properties of the hydrogel formulation. This additional polymer preferably is hydrophilic and may represent any type of polymer backbone known in the art, preferably polyethers, polyesters, polyamides, polyoxazolines, polyamines, polyacrylates, polymethacrylates, polyolefins, hydrogenated polyolefins, polysiloxanes, polycarbonates, (per)fluorinated polyethers, polyvinylenes, or copolymers of such polymers. More preferably, the polymer backbone is a polyether, polyester, polyacrylate, polymethacrylate, polyolefin, hydrogenated polyolefin, polycarbonate, polyvinylene, or a co-polymer of such polymers. Even more preferred are polyethers, polyesters, or copolymers thereof. Most preferably, this additional polymer is a polyether, preferably a polyglycol, preferably a polyethylene glycol or a poly ethylene-co-propylene glycol (random or block), most preferably a polyethylene glycol. Preferably, the additional polymer comprises at least one moiety *-A-* as defined hereinbefore in the context of the supramolecular polymer according to the first aspect.

The hydrogel formulation may comprise as further ingredients one or more selected from a solid filler, a diluent, a thickener, a carrier, a pH buffer and other excipients known in the art. A non-limited list of further ingredients includes (fluorescent) dyes, contrast agents, sugars, starches, cellulose and its derivatives, gelatin, talc, clays, laponite particles, bentonite particles, waxes (natural and synthetic), oils from natural origin, fatty acids and their esters, and ionic additives. Preferably, pH buffers are added as further ingredient to improve the hydrogel properties. The pH-buffer may be selected from buffers comprising acetic acid, citric acid, boric acid, phosphate salts, Tris, Tricine, Bicine, TAPS, TAPSO, HEPES, PIPES, MES or TES. More preferably, the pH-buffer is phosphate buffered saline (PBS).

In another embodiment the hydrogel formulation comprises a pH-buffer that has a pKₐ between 5 and 8, preferably between 6 and 8, and most preferably between 7 and 8, wherein the pKₐ is equal to -log(Kₐ) and Kₐ is the acid dissociation constant.

In another embodiment, the hydrogel formulation comprises as further ingredients one or more biologically active and/or pharmaceutically active compounds. The inventors have found that the hydrogel formulation in gelled state can act as a carrier material for biologically active and/or pharmaceutically active compounds that can provide controlled or prolonged release of the biologically active and/or pharmaceutically active compounds to the environment.

In a very preferred embodiment, the hydrogel formulation comprises as further ingredients one or more pharmaceutically active ingredients.

A biologically active or pharmaceutically active compound, as used herein, includes a compound which provides a therapeutic, diagnostic or prophylactic effect, a compound that affects or participates in tissue growth, cell growth, cell differentiation, a compound that may be able to invoke a biological action such as an immune response, or a compound that could play any other role in one or more biological processes. Such compounds, peptide or non-peptide, protein or non-protein, organic or inorganic, include but are not limited to bone morphogenetic proteins, antimicrobial agents (including antibacterial and anti-fungal agents), anti-viral agents, anti-tumor agents, chemotherapeutic agents, hormones, hormone antagonistics, corticosteroids such as mineralocorticosteroids or glucocorticosteroids, androgents, estrogens, progestins immunogenic agents, anti-inflammatory agents, anti-gout agents, centrally acting analgesics, local anesthetics, centrally active muscle relaxants, paracrine factors, growth factors, nucleic acids, DNA-derivatives, RNA-derivatives, lipids, lipopolysaccharides, (poly)saccharides, vitamins, alkali salts, phosphate salts, and peptides, polypeptides and proteins in general, preferably include but are not limited to bone morphogenetic proteins, antimicrobial agents (including antibacterial and anti-fungal agents), anti-viral agents, anti-tumor agents, chemotherapeutic agents, hormones, hormone antagonistics, corticosteroids such as mineralocorticosteroids or glucocorticosteroids, androgents, estrogens, progestins immunogenic agents, anti-inflammatory agents, anti-gout agents, growth factors, nucleic acids, DNA-derivatives, RNA-derivatives, lipids, lipopolysaccharides, (poly)saccharides, alkali salts, phosphate salts and peptides, polypeptides and proteins in general, more preferably, antimicrobial agents (including antibacterial and anti-fungal agents), anti-viral agents, anti-tumor agents and chemotherapeutic agents, most preferably anti-tumor agents and chemotherapeutic agents.

The hydrogel formulation preferably comprises 0.0001 to 30 wt.% of one or more biologically active and/or one or more pharmaceutically active compounds, such as the one or more biologically active and/or one or more pharmaceutically active compounds as defined hereinbefore, more preferably 0.01 to 10 wt.%, and most preferably 0.1 to 5 wt.%, based on the weight of the hydrogel formulation.

In a preferred embodiment, the hydrogel formulation comprises one or more biologically active and/or one or more pharmaceutically active compounds as the one or more further ingredients, selected from the group consisting of peptides, proteins, antimicrobial agents (including antibacterial and anti-fungal agents), anti-viral agents, anti-tumor agents, chemotherapeutic agents, corticosteroids, alkali salts, phosphate salts, local anesthetics and combinations thereof, preferably in an amount of 0.0001 to 30 wt.%, based on the weight of the hydrogel formulation, more preferably 0.01 to 10 wt.%, and most preferably 0.1 to 5 wt.%.

Preferably, the hydrogel formulation comprises one or more biologically active and/or one or more pharmaceutically active compounds as the one or more further ingredients which provide a therapeutic, diagnostic or prophylactic effect, or inhibit cell growth or differentiation, selected from the group consisting of peptides, proteins, antimicrobial agents (including antibacterial and anti-fungal agents), anti-viral agents, hormones, anti-tumor agents, chemotherapeutic agents, anti-inflammatory drugs, corticosteroids, local anesthetics and combinations thereof, preferably in an amount of 0.0001 to 30 wt.%, based on the weight of the hydrogel formulation, more preferably 0.01 to 10 wt.%, and most preferably 0.1 to 5 wt.%.

In a very preferred embodiment, the hydrogel formulation comprises 0.0001 to 30 wt.% of one or more pharmaceutically active ingredients, based on the weight of the hydrogel formulation. Preferably, the hydrogel formulation comprises one or more pharmaceutically active compounds as the one or more further ingredients, selected from the group consisting of anti-tumor agents, chemotherapeutic agents, local anesthetics and combinations thereof.

In another very preferred embodiment, the hydrogel formulation comprises one or more pharmaceutically active compounds as the one or more further ingredients selected from the group consisting of antimicrobial agents (including antibacterial and anti-fungal agents), anti-viral agents, anti-tumor agents, chemotherapeutic agents, corticosteroids, alkali salts, phosphate salts, local anesthetics and combinations thereof, preferably in an amount of 0.0001 to 30 wt.%, based on the weight of the hydrogel formulation, more preferably 0.01 to 10 wt.%, and most preferably 0.1 to 5 wt.%.

In an embodiment, the hydrogel formulation comprises 0.01 to 2 wt.%, more preferably 0.02 to 1 wt.% of one or more pharmaceutically active ingredients selected from the group consisting of alkali salts, phosphate salts, and combinations thereof, based on the weight of the hydrogel formulation.

Preferred examples of anti-inflammatory drugs include non-steroidal anti-inflammatory drugs. Preferred examples of corticosteroids include glucocorticosteroids. Preferred examples of local anesthetics include local anesthetics with an amide group, such as lidocaine, bupivacaine and levobupivacaine.

It is possible within the scope of the present invention to incorporate drugs of a polymeric nature, but also to incorporate drugs or vitamins of a relatively small molecular weight of less than about 1500 Da, or even less than about 500 Da.

The inventors have unexpectedly established that the hydrogel formulations comprising the supramolecular polymer according to the invention behave liquid-like at a pH which is between 8.5 and 14.0, such as between 9.0 and 11.0, and at a temperature of between 20 and 40 °C. The corresponding dynamic viscosity at these conditions is low enough to inject the hydrogel formulations using for example a syringe equipped with a needle or a catheter. Moreover, they unexpectedly found that the hydrogel formulations comprising the supramolecular polymer according to the invention behave solid-like at a pH between 2.0 and less than 8.0 and at a temperature of 37 °C. At a pH between 2.0 and less than 8.0, such as between 3.0 and 7.5, and at a temperature of 37 °C, a gel is obtained with an unexpectedly high mechanical strength, even at low concentrations of the supramolecular polymer and without the need to chemically crosslink the polymer chains. Hence, the hydrogel formulations comprising the supramolecular polymer according to the invention can be switched between a liquid state and a gelled state using the pH of the hydrogel formulation and hydrogels are formed already at low concentrations of the supramolecular polymer and at a wide range of temperatures, thereby resulting in stable, yet injectable, hydrogels with favorable mechanical performances that are eminently suitable for applications such as drug delivery, barrier films, absorbents, anti-adhesion films and (dermal) fillers.

Without wishing to be bound by any theory, it is believed that this unique combination of rheological properties is caused by the occurrence of a relatively large number of blocks of the following repeating units *-[R³-L-A-L]-*, such as for example *-R³-L-A-L-R³-L-A- L- *, in the polymer chains as well as the presence of relatively long chains of linear hydrophilic polymeric groups *-POL-* in the polymer chains. At basic (increased) pH, the hydrogen-bonding units in the repeating units *-[R³-L-A-L]-* are deprotonated and the polymer chains solubilize, with help of the linear hydrophilic polymeric groups. At lower pH, such as neutral pH, strong hydrogen bonds develop between the repeating units *-[R³-L-A-L]- * and a hydrogel with solid-like properties is formed.

In a preferred embodiment, the hydrogel formulation has a pH between 8.5 and 14.0, preferably a pH between 9.0 and 10.0, and is a liquid at a temperature of between 20 and 40 °C.

In a preferred embodiment, the hydrogel formulation has a pH between 8.5 and 14.0, preferably a pH between 9.0 and 10.0, and is a liquid at a temperature of between 20 and 40 °C and at a concentration of 5 wt.% of the supramolecular polymer, based on the weight of the hydrogel formulation.

In a very preferred embodiment, the hydrogel formulation has a pH between 8.5 and 14.0, preferably a pH between 9.0 and 10.0, is a liquid at a temperature of between 20 and 40 °C and has a dynamic viscosity at 37 °C of between 0.01 and 20 Pa·s, as measured with a rheometer with a plate-plate geometry at a shear rate of 1 s⁻¹ and with a gap distance of 0.50 mm.

In a very preferred embodiment, the hydrogel formulation has a pH between 8.5 and 14.0, preferably a pH between 9.0 and 10.0, is a liquid at a temperature of between 20 and 40 °C and at a concentration of 5 wt.% of the supramolecular polymer, based on the weight of the hydrogel formulation, and has a dynamic viscosity at 37 °C and at a concentration of 5 wt.% of the supramolecular polymer, based on the weight of the hydrogel formulation, of between 0.01 and 20 Pa·s, as measured with a rheometer with a plate-plate geometry at a shear rate of 1 s⁻¹ and with a gap distance of 0.50 mm.

In a very preferred embodiment, the hydrogel formulation has a pH between 8.5 and 14.0, preferably a pH between 9.0 and 10.0, is a liquid at a temperature of between 20 and 40 °C and has a dynamic viscosity at 37 °C of between 0.05 and 10 Pa·s, as measured with a rheometer with a plate-plate geometry at a shear rate of 1 s⁻¹ and with a gap distance of 0.50 mm.

In a very preferred embodiment, the hydrogel formulation has a pH between 8.5 and 14.0, preferably a pH between 9.0 and 10.0, is a liquid at a temperature of between 20 and 40 °C and at a concentration of 5 wt.% of the supramolecular polymer, based on the weight of the hydrogel formulation, and has a dynamic viscosity at 37 °C and at a concentration of 5 wt.% of the supramolecular polymer, based on the weight of the hydrogel formulation, of between 0.05 and 10 Pa·s, as measured with a rheometer with a plate-plate geometry at a shear rate of 1 s⁻¹ and with a gap distance of 0.50 mm.

In a very preferred embodiment, the hydrogel formulation has, at a pH between 2.0 and less than 8.0, preferably at a pH between 3.0 and 7.5:
(i) storage moduli *G*' of at least 20 Pa, preferably at least 200 Pa, most preferably at least 2000 Pa across a frequency range of 0.2 to 20 Hz; and/or
(ii) tan(*δ*) values of lower than 0.2, preferably between 0.05 and 0.15, across a frequency range of 0.2 to 20 Hz,
wherein the storage moduli *G*' and tan(*δ*) values are measured with a rheometer with a plate-plate geometry and a gap distance of about 0.50 mm, at oscillatory frequencies between 0.2 and 20 Hz and at a temperature of 37 °C.

In a very preferred embodiment, the hydrogel formulation has, at a pH between 2.0 and less than 8.0, preferably at a pH between 3.0 and 7.5, and at a concentration of 5 wt.% of the supramolecular polymer, based on the weight of the hydrogel formulation:
(i) storage moduli G' of at least 20 Pa, preferably between 20 and 1000 Pa, across a frequency range of 0.2 to 20 Hz; and
(ii) tan(*δ*) values of lower than 0.2, preferably between 0.05 and 0.15, across a frequency range of 0.2 to 20 Hz,
wherein the storage moduli G' and tan(*δ*) values are measured with a rheometer with a plate-plate geometry and a gap distance of about 0.50 mm, at oscillatory frequencies between 0.2 and 20 Hz and at a temperature of 37 °C.

In a very preferred embodiment, the hydrogel has, at a pH between 2.0 and less than 8.0, preferably at a pH between 3.0 and 7.5, and at a concentration of 5 wt.% of the supramolecular polymer, based on the weight of the hydrogel formulation, storage moduli *G'* which are larger than the loss moduli *G"* until at least 100 % deformation, preferably until at least 300 % deformation, in a strain sweep measurement, wherein the storage moduli *G'* and loss moduli *G"* values are measured with a rheometer with a plate-plate geometry and a gap distance of about 0.50 mm, at an oscillatory frequency of 1 Hz and at a temperature of 37 °C.

In a very preferred embodiment, the hydrogel formulation has, at a pH between 2.0 and less than 8.0, preferably at a pH between 3.0 and 7.5, and at a concentration of 5 wt.% of the supramolecular polymer, based on the weight of the hydrogel formulation, storage moduli *G'* which are larger than the loss moduli *G"* at all temperatures between 20 °C and 45 °C, preferably at all temperatures between 20 °C and 50 °C, most preferably at all temperatures between 20 °C and 60 °C, wherein the storage moduli *G'* and loss moduli *G"* values are measured with a rheometer with a plate-plate geometry and a gap distance of about 0.50 mm, at an oscillatory frequency of 1 Hz.

The term *'tan(δ)',* wherein *δ* is the phase shift, is defined by the ratio *G"*/*G',* as is commonly known in the field of rheology. *G"* represent the loss modulus and characterizes the viscous character or the liquid-like behavior of the hydrogel formulation. *G'* represents the storage modulus and characterizes the elastic character or the solid-like behavior of the hydrogel formulation. If a hydrogel formulation shows purely viscous (liquid-like) behavior and there is no elastic behavior, *δ*=90°, *G'=*0 and tan(*δ*)=∞. If a hydrogel formulation shows purely elastic (solid-like) behavior and there is no viscous behavior, *δ*=0°, *G"=0* and tan(*δ*)=0. If the hydrogel formulation has a non-zero phase shift *δ* of lower than 45°, tan(*δ*) is lower than 1, G' is larger than G" and the hydrogel formulation shows gel-like behavior in the sense that elastic behavior dominates viscous behavior.

In a very preferred embodiment, the hydrogel formulation has elastic behavior at a pH between 2.0 and less than 8.0, preferably at a pH between 3.0 and 7.5, and at a temperature of 20 °C, wherein said elastic behavior is characterized by:
(i) a Young's modulus (E_{mod}) of at least 0.05 MPa, preferably at least 0.1 MPa, even more preferably of at least 1 MPa, as determined by test method ASTM D 1708-96 with a crosshead speed of 20 mm/min, preferably measured between 0.25 and 2.50% elongation; and/or
(ii) a modulus at 100% elongation of at least 0.03 MPa, more preferably at least 0.1 MPa, most preferably at least 0.5 MPa, as determined by test method ASTM D 1708-96 with a crosshead speed of 20 mm/min; and/or
(iii) an ultimate tensile strength of at least 0.15 MPa, more preferably of at least 1 MPa, and most preferably of at least 15 MPa, as determined by test method ASTM D 1708-96 with a crosshead speed of 20 mm/min; and/or
(iv) an elongation at break of at least 10%, more preferably of at least 150%, and most preferably of at least 250%, as determined by test method ASTM D 1708-96 with a crosshead speed of 20 mm/min.

In a very preferred embodiment, the hydrogel formulation has elastic behavior at a pH between 2.0 and less than 8.0, preferably at a pH between 3.0 and 7.5, and at a temperature of 20 °C, wherein said elastic behavior is characterized by at least two, preferably by all of the elastic properties (i) to (iv) listed above.

In a very preferred embodiment, the hydrogel formulation, at a pH between 2.0 and less than 8.0, preferably at a pH between 3.0 and 7.5, is in a gelled state after submerging the supramolecular polymer in phosphate buffered saline (PBS) for 24 h at a temperature of 37 °C, whereby the amount of PBS is equal to 40 times the weight of the supramolecular polymer, more preferably the hydrogel formulation has, at a pH between 2.0 and less than 8.0, a tan(*δ*) value lower than 1 across a frequency range of 0.2 to 20 Hz, most preferably lower than 0.8, after submerging the supramolecular polymer in phosphate buffered saline (PBS) in an amount of PBS equal to 40 times the weight of the supramolecular polymer, for 24 h at a temperature of 37 °C, wherein the tan(*δ*) values are measured with a rheometer with a plate-plate geometry and a gap distance of 0.50 mm, at oscillatory frequencies between 0.2 and 20 Hz and at a temperature of 37 °C.

### Medical uses and treatments

In a fifth aspect, the invention concerns a hydrogel formulation according to the fourth aspect having a pH between 8.5 and 14.0, preferably a pH between 9.0 and 10.0, which is a liquid at a temperature of between 20 and 40 °C, and comprising 0.0001 to 30 wt.% of one or more pharmaceutically active ingredients, based on the weight of the hydrogel formulation, for use in the treatment of oncological diseases, cardio-vascular diseases, orthopaedic diseases, gastrointestinal diseases or wound care in mammalian subjects, said treatment comprising injecting the hydrogel formulation into the mammalian body, followed by release of the one or more pharmaceutically active ingredients from the hydrogel formulation.

The fifth aspect can also be worded as a method of treatment of oncological diseases, cardio-vascular diseases, orthopaedic diseases, gastrointestinal diseases or wound care in mammalian subjects, said method comprising injecting a hydrogel formulation according to the fourth aspect having a pH between 8.5 and 14.0, preferably a pH between 9.0 and 10.0, which is a liquid at a temperature of between 20 and 40 °C, and comprising 0.0001 to 30 wt.% of one or more pharmaceutically active ingredients, based on the weight of the hydrogel formulation, into the mammalian body, followed by release of the one or more pharmaceutically active ingredients from the hydrogel formulation.

In a sixth aspect, the invention concerns a hydrogel formulation according to the fourth aspect having a pH between 8.5 and 14.0, preferably a pH between 9.0 and 10.0, which is a liquid at a temperature of between 20 and 40 °C, and comprising 0.0001 to 30 wt.% of one or more pharmaceutically active ingredients, based on the weight of the hydrogel formulation, for use in a method of prevention of tissue adhesion or in reconstructive surgery or cosmetic surgery in mammalian subjects, said method comprising injecting the hydrogel formulation into the mammalian body, followed by release of the one or more pharmaceutically active ingredients from the hydrogel formulation.

The sixth aspect can also be worded as a method of prevention of tissue adhesion or reconstructive surgery or cosmetic surgery in mammalian subjects, said method comprising injecting a hydrogel formulation according to the fourth aspect having a pH between 8.5 and 14.0, preferably a pH between 9.0 and 10.0, which is a liquid at a temperature of between 20 and 40 °C, and comprising 0.0001 to 30 wt.% of one or more pharmaceutically active ingredients, based on the weight of the hydrogel formulation, into the mammalian body, followed by release of the one or more pharmaceutically active ingredients from the hydrogel formulation.

In an embodiment, the injection is performed through a syringe equipped with a needle, a double chamber syringe equipped with a needle, a catheter, or by spraying or pumping.

As explained hereinbefore, the hydrogel formulation comprising the supramolecular polymer according to the invention behaves solid-like at a pH between 2.0 and less than 8.0 and at a temperature of 37 °C. Hence, after injection or spraying of the hydrogel formulation into or onto the mammalian body, a gel is formed at neutral or slightly acidic pH (~5.0 - 7.5, depending on the specific tissue) due to the buffering capacity of the body. The hydrogel formulation can then act as a reservoir for the one or more pharmaceutically active ingredients. The inventors have established that the reservoir can provides prolonged and/or continuous release of the one or more pharmaceutically active ingredients to the body.

In a seventh aspect, the invention concerns a hydrogel formulation according to the fourth aspect having a pH between 2.0 and less than 8.0, which is a gel at a temperature of between 20 and 40 °C, and comprising 0.0001 to 30 wt.% of one or more pharmaceutically active ingredients, based on the weight of the hydrogel formulation, for use in a method of treatment or prevention of bacterial or viral infections in a mammal, said method comprising applying the hydrogel formulation onto the mammalian body, preferably onto the skin of the mammalian body.

The hydrogel formulation can be used in a method of treatment or prevention of bacterial or viral infections in a mammal, said method comprising applying a hydrogel formulation according to the fourth aspect having a pH between 2.0 and less than 8.0, which is a gel at a temperature of between 20 and 40 °C, and comprising 0.0001 to 30 wt.% of one or more pharmaceutically active ingredients, based on the weight of the hydrogel formulation onto the mammalian body, preferably onto the skin of the mammalian body.

Preferred embodiments defined in the context of the fourth aspect are equally applicable to the fifth aspect, to the sixth aspect and to the seventh aspect.

### EXAMPLES

### Example 1: Preparation of Polymer 1

Telechelic hydroxy terminated poly(ethylene glycol) with a molecular weight of 20 kDa (20.0 gram, 1.00 mmol) was dried at 110 °C in vacuo for 2 hours. Subsequently, 5(2-hydroxyethyl)-6-methyl isocytosine (338 mg, 2.00 mmol), hexanediisocyanate (1.01 gram, 3.00 mmol), 50 mL dimethylformamide and one drop of dibutyltin(IV)dilaurate were added to the polymer. The reaction mixture was stirred for 12 hours at 90 °C. Subsequently, the reaction mixture was diluted with 50 mL of methanol and poured into 500 mL of diethylether. The precipitated polymer was dissolved into 70 mL chloroform and 70 mL methanol and poured into 500 mL diethylether. The precipitated polymer was dried in vacuo and obtained as a white solid. SEC (DMF/LiBr, PEO-standards): Mₙ = 88 kDa.

### Example 2: Preparation of Polymer 2

Telechelic hydroxy terminated poly(ethylene glycol) with a molecular weight of 20 kDa (43.5 gram, 2.18 mmol) was dried at 110 °C in vacuo for 2 hours. Subsequently, 5(2-hydroxyethyl)-6-methyl isocytosine (553 mg, 3.27 mmol), 4,4'-methylene-bis(cyclohexyl isocyanate) (1.42 gram, 5.43 mmol), 50 mL dimethylformamide and one drop of dibutyltin(IV)dilaurate were added to the polymer. The reaction mixture was stirred for 12 hours at 90 °C. Subsequently, the reaction mixture was diluted with 100 mL of methanol and poured into 1000 mL of diethylether. The precipitated polymer was dissolved into 70 mL chloroform and 70 mL methanol and poured into 1000 mL diethylether. The precipitated polymer was dried in vacuo and obtained as a white solid. SEC (DMF/LiBr, PEO-standards): Mₙ = 62 kDa.

### Example 3: Preparation of Polymer 3

Telechelic hydroxy terminated poly(ethylene glycol) with a molecular weight of 20 kDa (20.0 gram, 1.00 mmol) was dried at 110 °C in vacuo for 2 hours. Subsequently, 5(2-hydroxyethyl)-6-methyl isocytosine (338 mg, 2.00 mmol), isophoronediisocyanate (0.70 gram, 3.15 mmol), 50 mL dimethylformamide and one drop of dibutyltin(IV)dilaurate were added to the polymer. The reaction mixture was stirred for 12 hours at 90 °C. Subsequently, the reaction mixture was diluted with 50 mL of methanol and poured into 500 mL of diethylether. The precipitated polymer was dissolved into 70 mL chloroform and 70 mL methanol and poured into 500 mL diethylether. The precipitated polymer was dried in vacuo and obtained as a white solid. SEC (DMF/LiBr, PEO-standards): Mₙ = 80 kDa.

### Example 4: Preparation of Polymer 4

Telechelic hydroxy terminated poly(ethylene glycol) with a molecular weight of 10 kDa (20.0 gram, 2.00 mmol) was dried at 110 °C in vacuo for 2 hours. Subsequently, 5(2-hydroxyethyl)-6-methyl isocytosine (676 mg, 4.00 mmol), isophoronediisocyanate (1.40 gram, 6.31 mmol), 50 mL dimethylformamide and one drop of dibutyltin(IV)dilaurate were added to the polymer. The reaction mixture was stirred for 12 hours at 90 °C. Subsequently, the reaction mixture was diluted with 50 mL of methanol and poured into 500 mL of diethylether. The precipitated polymer was dissolved into 70 mL chloroform and 70 mL methanol and poured into 500 mL diethylether. The precipitated polymer was dried in vacuo and obtained as a white solid. SEC (DMF/LiBr, PEO-standards): Mₙ = 53 kDa.

### Example 5: Preparation of Polymer 5

Telechelic hydroxy terminated poly(ethylene glycol) with a molecular weight of 20 kDa (20.0 gram, 1.00 mmol) was dried at 110 °C in vacuo for 2 hours. Subsequently, 5(2-hydroxyethyl)-6-methyl isocytosine (507 mg, 3.00 mmol), hexanediisocyanate (0.671 gram, 4.00 mmol), 50 mL dimethylformamide and one drop of dibutyltin(IV)dilaurate were added to the polymer. The reaction mixture was stirred for 12 hours at 90 °C. Subsequently, the reaction mixture was diluted with 50 mL of methanol and poured into 500 mL of diethylether. The precipitated polymer was dissolved into 70 mL chloroform and 70 mL methanol and poured into 500 mL diethylether. The precipitated polymer was dried in vacuo and obtained as a white solid. SEC (DMF/LiBr, PEO-standards): Mₙ = 62 kDa.

### Example 6: Preparation of Polymer 6

Telechelic hydroxy terminated poly(ethylene glycol) with a molecular weight of 6 kDa (21.9 gram, 3.65 mmol) was dried at 110 °C in vacuo for 2 hours. Subsequently, 5(2-hydroxyethyl)-6-methyl isocytosine (1.231 g, 7.28 mmol), hexanediisocyanate (1.85 gram, 11.0 mmol), 30 mL dimethylformamide and one drop of dibutyltin(IV)dilaurate were added to the polymer. The reaction mixture was stirred for 12 hours at 90 °C. Subsequently, the reaction mixture was diluted with 50 mL of methanol and poured into 500 mL of diethylether. The precipitated polymer was dissolved into 70 mL chloroform and 70 mL methanol and poured into 500 mL diethylether. The precipitated polymer was dried in vacuo and obtained as a white solid. SEC (DMF/LiBr, PEO-standards): Mₙ = 24 kDa

### Example 7: Preparation of Polymer 7

Telechelic hydroxy terminated poly(ethylene glycol) with a molecular weight of 4 kDa (20.0 gram, 5.00 mmol) was dried at 110 °C in vacuo for 2 hours. Subsequently, 5(2-hydroxyethyl)-6-methyl isocytosine (1.69 g, 10.0 mmol), isophoronediisocyanate (3.56 gram, 16.0 mmol), 30 mL dimethylformamide and one drop of dibutyltin(IV)dilaurate were added to the polymer. The reaction mixture was stirred for 12 hours at 90 °C. Subsequently, the reaction mixture was diluted with 50 mL of methanol and poured into 500 mL of diethylether. The precipitated polymer was dissolved into 70 mL chloroform and 70 mL methanol and poured into 500 mL diethylether. The precipitated polymer was dried in vacuo and obtained as a white solid. SEC (DMF/LiBr, PEO-standards): Mₙ = 16 kDa.

### Comparative Example 1: Polymer C1

Polymer C1 is a polymer as disclosed in EP1972661A1 comprising PEG 10000 and urea linkers. The polymer was obtained as described in EP1972661A1 for water gellant 8B from prepolymer10K-10.

### Comparative Example 2: Polymer C2

Polymer C2 is a polymer as disclosed in EP1907482A1 comprising PEG10000 whereby the molar ratio of compound A' to HO-POL-OH is 1.0 : 1.0 and i = 1 (i as defined in Formula (I) according to the first aspect of this invention). The polymer was obtained as described in EP1907482A1, Example 8, with PEG10000 and 1 mole equivalent of UPy2.

### Comparative Example 3: Polymer C3 and its hydrogel

Polymer C3 is a polymer as disclosed in EP1972661A1 comprising PEG6000 whereby the molar ratio of compound A' to HO-POL-OH is 1.0 : 1.0 and i = 1 (i as defined in Formula (I) according to the first aspect of this invention). The polymer was obtained as described in EP1972661A1, Example 9, by reacting aminohexyl end-capped PEG6000 with 1 mole equivalent of UPy2. The resulting polymer was brought in water resulting in an elastic hydrogel at 5% solids loading. Subsequent addition of 1 N NaOH (aq) solution to pH = 9 followed by heating to 70 °C did not lead to liquid formulation, nor could the mixture be injected with a syringe equipped with a 18G needle at room temperature.

### Example 8: Preparation of Hydrogel formulation 1

Polymer 1 prepared in Example 1 was used to prepare a hydrogel formulation. The hydrogel formulation comprised 95 wt.% of water, 5 wt.% of the supramolecular polymer, to which around 1 mole equivalent of NaOH to compound A' was added by adding the desired amount of 1N NaOH(aq) solution. The resulting mixture was subsequently stirred at 70 °C until a homogeneous solution was obtained. The pH of the resulting hydrogel formulation was around 9. This hydrogel formulation was a liquid and could be injected with a syringe equipped with a 18G needle. Injecting this formulation directly into a 40x larger volume of PBS directly resulted in the formation of a hydrogel.

### Example 9: Temperature stability of hydrogel formulations

Hydrogel formulations for rheological measurements were obtained by dissolving the supramolecular polymer into ethanol/water (85/15 V/V) mixtures at 10 wt.% solids by stirring at 60 °C until a homogeneous solution was obtained. This solution was poured into a Teflon mould and allowed to dry resulting in a solid film of the polymer. To the resulting film was added the PBS (pH = 7.2) to get the hydrogel formulation at the desired wt.%. From this hydrogel specimens were cut for use in the rheometer.

The storage moduli *G*' and loss moduli *G"* were measured with a rheometer with a plate-plate geometry and a gap distance of about 0.50 mm, at oscillatory frequency of 1 Hz and a strain of 1% with a temperature sweep from 20 °C to 60 °C. The results at temperatures of 20, 45 and 60 °C are depicted in Table 1.

**Table 1: Temperature stability hydrogel formulations**

| Supramolecular polymer | wt.% in PBS | G' (Pa) @20°C | G" (Pa) @20°C | G' (Pa) @45°C | G" (Pa) @45°C | G' (Pa) @50°C | G" (Pa) @50°C | G' (Pa) @60°C | G" (Pa) @60°C |
|---|---|---|---|---|---|---|---|---|---|
| Example 1 | 5% | 60 | 3.5 | 50 | 3.5 | 40 | 3.4 | 10 | 1.2 |
| Example 1 | 10% | 320 | 18 | 220 | 20 | 200 | 18 | 90 | 12 |
| Example 2 | 5% | 610 | 130 | 210 | 96 | 130 | 70 | 44 | 40 |
| Example 4 | 5% | 420 | 90 | 100 | 35 | 60 | 28 | 48 | 17 |
| Example 4 | 2.5% | 95 | 18 | 45 | 11 | 31 | 9 | 20 | 5 |
| Example 5 | 5% | 200 | 15 | 110 | 12 | 100 | 13 | 74 | 11 |

### Example 10: Deformation stability of hydrogel formulations

Hydrogel formulations for rheological measurements were obtained by dissolving the supramolecular polymer into ethanol/water (85/15 V/V) mixtures at 10 wt.% solids by stirring at 60 °C until a homogeneous solution was obtained. This solution was poured into a Teflon mould and allowed to fully dry resulting in a solid film of the polymer. To the resulting film was added the PBS (pH = 7.2) to get the hydrogel at 5 wt.%. From this hydrogel specimens were cut for use in the rheometer.

The storage moduli *G'* and loss moduli *G"* were measured with a rheometer with a plate-plate geometry and a gap distance of about 0.50 mm, at a temperature of 37 °C and an oscillatory frequency of 1 Hz and a strain sweep from 1 to 1000 %. The results are depicted for 100% and 300% deformation in Table 2.

**Table 2: Deformation stability hydrogel formulations in strain sweep**

| Supramolecular polymer | wt.% in PBS | G' (Pa) @100% | G" (Pa) @100% | G' (Pa) @300% | G" (Pa) @300% |
|---|---|---|---|---|---|
| Example 1 | 5 | 32 | 4.5 | 30 | 12 |
| Example 2 | 5 | 380 | 110 | 120 | 80 |
| Example 4 | 5 | 230 | 60 | 90 | 30 |
| Example 5 | 5 | 100 | 48 | 36 | 50 |

### Example 11: Tensile properties of hydrogel formulations

Hydrogel formulations for tensile measurements were obtained by dissolving the supramolecular polymer into ethanol/water (85/15 V/V) mixtures at 10 wt.% solids by stirring at 60 °C until a homogeneous solution was obtained. This solution was poured into a Teflon mould and allowed to fully dry resulting in a solid film of the polymer. To the resulting film was added the PBS (pH = 7.2) to get the hydrogel at the desired wt.%. From this hydrogel, specimen dog bones were cut for use in the tensile tester.

Tensile properties were determined by test method ASTM D 1708-96 with a crosshead speed of 20 mm/min at a temperature of 20 °C and the Young's modulus was measured between 0.25 and 2.50% elongation. The results are depicted in Table 3.

**Table 3: Tensile properties hydrogel formulations**

| Supramolecular polymer | wt.% in PBS | Young's modulus [MPa] | E100% [MPa] | Ultimate tensile strength [MPa] | Elongation at break [%] |
|---|---|---|---|---|---|
| Example 2 | 10 | 0.08 | 0.05 | 0.16 | 250 |
| Example 3 | 10 | 0.19 | 0.12 | 0.28 | 590 |
| Example 4 | 10 | 0.51 | 0.38 | 0.91 | 210 |

## Claims

1. Supramolecular polymer comprising polymer chains according to Formula (I): 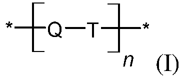 wherein:
• the average n in the supramolecular polymer is between 2 and 16, one of the building blocks Q is connected to a terminal group via the bond marked with an asterisk, and one of the building blocks T is connected to a terminal group via the bond marked with an asterisk;
• building block *-Q-* represents: 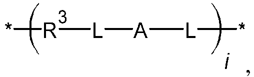 wherein the average i in the supramolecular polymer is between 1.5 and 6.0;
• building block *-T-* represents: 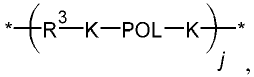 wherein the average j in the supramolecular polymer is between 1 and 6;
• the supramolecular polymer has an average molecular weight Mₙ of about 15 kDa to about 150 kDa, as determined with size-exclusion chromatography (SEC) with a GPC-system using RI detection with DMF comprising 10 mM LiBr at 50 °C as eluent, with the SEC-data being relative to PEO/PEG-standards;
• POL is a linear hydrophilic polymeric group having an average molecular weight Mₙ of about 1 kDa to about 30 kDa;
• moiety A represents moieties selected from the group consisting of Formulas (II-A) to (II-F), tautomers thereof and combinations thereof, wherein A is connected to L via the bonds marked with an asterisk in Formulas (II-A) to (II-F): 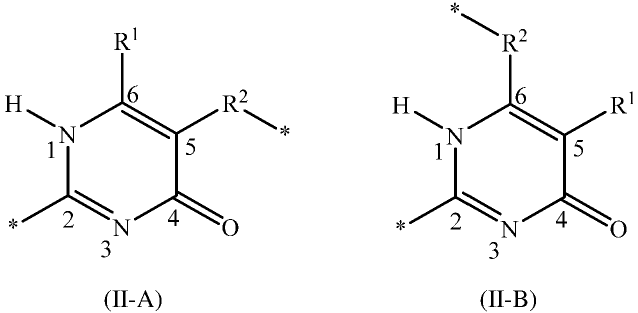 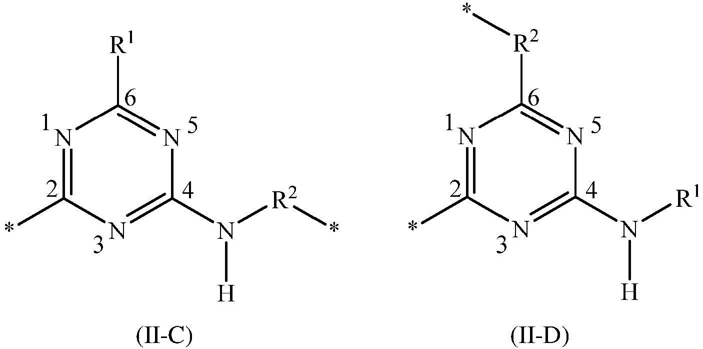 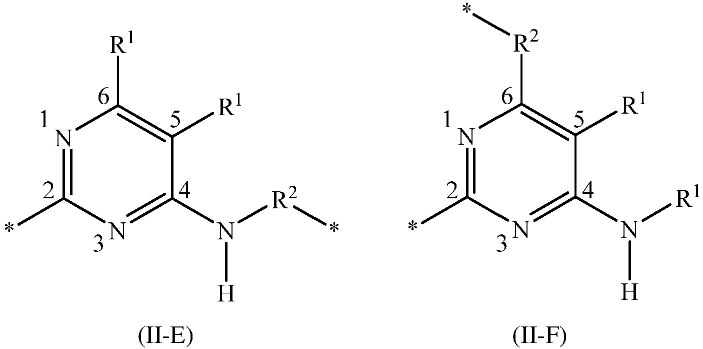
• each K is a urethane linking group;
• each L independently is a urethane or urea linking group, with the proviso that any moiety A according to Formula (II-A) and (II-B) is always coupled to a urea linking group L on the 2-position of the 4-pyrimidone, any moiety A according to Formula (II-C) and (II-D) is always coupled to a urea linking group L on the 2-position of the triazine, and any moiety A according to Formula (II-E) and (II-F) is always coupled to a urea linking group L on the 2-position of the pyrimidine;
• each R¹ is independently selected from the group consisting of hydrogen and C₁ - C₂₀ alkyl;
• R² is selected from the group consisting of C₁ - C₂₀ alkylene, optionally substituted with O or S; and
• R³ is selected from the group consisting of linear or branched C₂ - C₂₀ alkylene groups and cyclic C₃ - C₂₄ alkylene groups.

2. Supramolecular polymer according to claim 1, wherein A in formula (I) represents the following moieties selected from the group consisting of Formula (II-A): 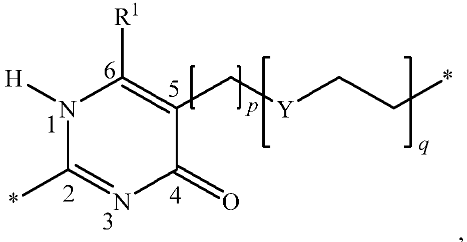 wherein:
R¹ is selected from the group consisting of hydrogen and C₁ - C₂₀ alkyl;
Y is O or S;
*p* is an integer of 1 to 20; and
*q* is an integer of 0 to 8,
preferably wherein R¹ is methyl and:
a) *p* is 2 and *q* is 0;
b) *p* is 2, *q* is 1 and Y is O; or
c) *p* is 4 to 11 and *q* is 0.

3. Supramolecular polymer according to claim 1 or 2, wherein:
• any moiety A according to Formula (II-A) and (II-B) is coupled to a urea linking group L on the 2-position of the 4-pyrimidone and to a urethane urea linking group L on the 5-position of the 4-pyrimidone;
• any moiety A according to Formula (II-C) and (II-D) is coupled to a urea linking group L on the 2-position of the triazine and to a urethane linking group L on the 4-position of the triazine,
• any moiety A according to Formula (II-E) and (II-F) is coupled to a urea linking group L on the 2-position of the pyrimidine and to a urethane linking group L on the 4-position of the pyrimidine.

4. Supramolecular polymer according to any one of claims 1 to 3, wherein R³ is selected from selected from hexylene,

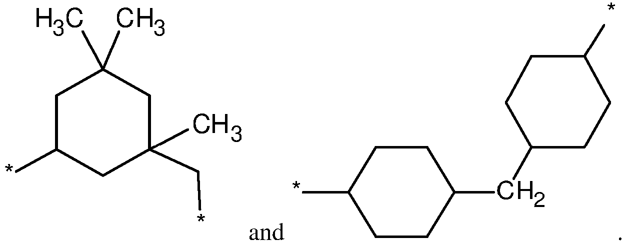

5. Supramolecular polymer according to any one of claims 1 to 4, wherein POL is a linear polyethylene glycol having an average molecular weight Mₙ of between 10 kDa and 25 kDa.

6. Process for the manufacture of a supramolecular polymer by reacting, optionally in a non-reactive solvent, a compound A' selected from the group consisting of Formulas (III-A) to (III-F), tautomers thereof and combinations thereof with a diisocyanate compound C' according to the Formula O=C=N-R³-N=C=O and a polymer HO-POL-OH: 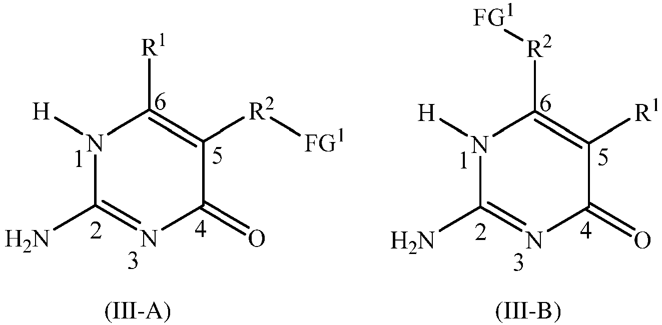 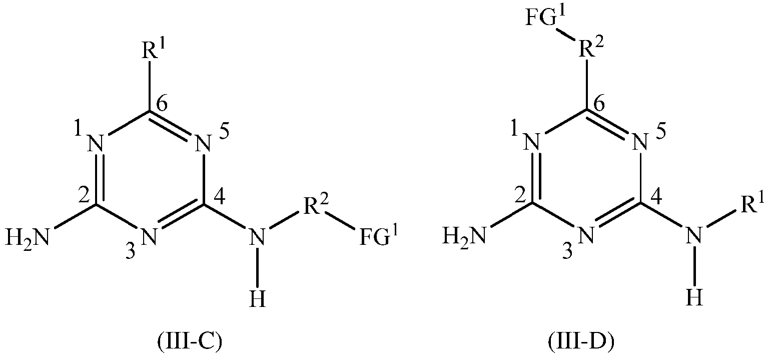 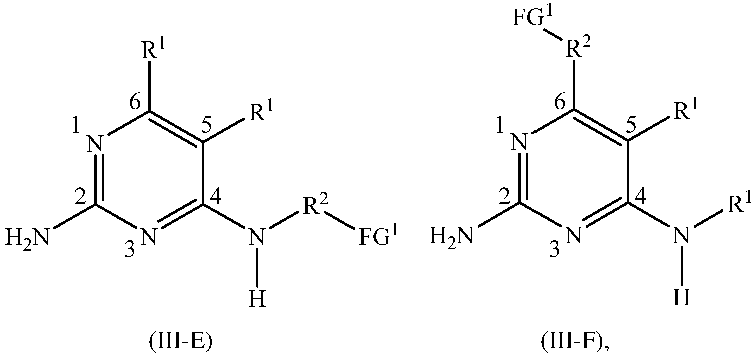
wherein R¹, R², R³ and POL are as defined in any one of claims 1 to 6,
wherein FG¹ represents a functional group selected from OH and NH₂, preferably OH, wherein the molar ratio of compound A' to HO-POL-OH applied during the reaction is between 1.5 : 1.0 and 6.0 : 1.0, and wherein the molar ratio of compound C' to the sum of compound A' and HO-POL-OH applied during the reaction is between 1.1 : 1.0 and 0.9 : 1.0.

7. Process according to claim 6, wherein A' represents moieties selected from the group consisting of Formula (III-A): 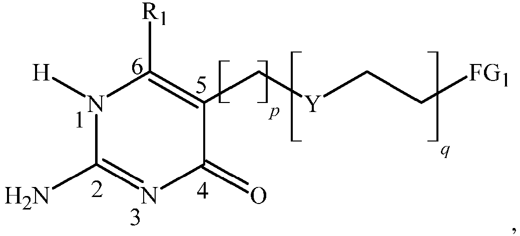 wherein:
R¹ is selected from the group consisting of hydrogen and C₁ - C₂₀ alkyl;
Y is O or S;
*p* is an integer of 1 to 20; and
*q* is an integer of 0 to 8,
preferably wherein R¹ is methyl and:
a) *p* is 2, *q* is 0 and FG¹ is OH;
b) *p* is 2, *q* is 1, Y is O and FG¹ is OH;
c) *p* is 4 to 11, *q* is 0 and FG¹ is OH; or
d) *p* is 4 to 11, *q* is 0 and FG¹ is NH₂.

8. Process according to claim 6 or 7, wherein molar ratio of compound A' to HO-POL-OH is between 1.5 : 1.0 and 4.0 : 1.0, and/or wherein the supramolecular polymer produced is a supramolecular polymer according to Formula (I) as defined in any one of claims 1-5.

9. Supramolecular polymer obtainable by the process according to claim 7 or 8.

10. Hydrogel formulation comprising 50.0 - 99.7 wt.% of water, 0.3 - 50.0 wt.% of the supramolecular polymer according to any one of claims 1 to 5 or 9, and 0 - 30 wt.% of further ingredients, based on the weight of the hydrogel formulation, wherein the amounts of water, supramolecular polymer and further ingredients add up to 100 wt.% of the hydrogel formulation.

11. Hydrogel formulation according to claim 10, which comprises 0.9 - 10.0 wt.% of the supramolecular polymer according to any one of claims 1 to 5 or 9, based on the weight of the hydrogel formulation.

12. Hydrogel formulation according to claim 10 or 11, which has a pH between 8.5 and 14.0 and is a liquid at a temperature between 20 and 40 °C, preferably having a pH between 8.5 and 14.0 and having a dynamic viscosity at 37 °C of between 0.01 and 20 Pa·s, as measured with a rheometer with a plate-plate geometry at a shear rate of 1 s⁻¹ and with a gap distance of 0.50 mm.

13. Hydrogel formulation according to claims 10 or 11, having, at a pH between 2.0 and less than 8.0:
(i) storage moduli *G*' of at least 20 Pa, preferably at least 200 Pa, most preferably at least 2000 Pa, across a frequency range of 0.2 to 20 Hz; and/or
(ii) tan(*δ*) values of lower than 0.2, preferably between 0.05 and 0.15, across a frequency range of 0.2 to 20 Hz,
wherein the storage moduli *G*' and tan(*δ*) values are measured with a rheometer with a plate-plate geometry and a gap distance of 0.50 mm, at oscillatory frequencies between 0.2 and 20 Hz and at a temperature of 37 °C.

14. Hydrogel formulation according to any of claims 10 - 13, which comprises 0.0001 to 30 wt.% of one or more pharmaceutically active ingredients, based on the weight of the hydrogel formulation.

15. Hydrogel formulation according to claim 12, comprising 0.0001 to 30 wt.% of one or more pharmaceutically active ingredients, based on the weight of the hydrogel formulation, for use in the treatment of oncological diseases, cardio-vascular diseases, orthopaedic diseases, gastrointestinal diseases or wound care in mammalian subjects, said treatment comprising injecting the hydrogel formulation into the mammalian body, followed by release of the one or more pharmaceutically active ingredients from the hydrogel formulation, preferably wherein the one or more pharmaceutically active ingredients are selected from the group consisting of anti-tumor agents, chemotherapeutic agents, local anesthetics and combinations thereof.

16. Hydrogel formulation according to claim 12, comprising 0.0001 to 30 wt.% of one or more pharmaceutically active ingredients, based on the weight of the hydrogel formulation, for use in a method of prevention of tissue adhesion or in reconstructive surgery or cosmetic surgery in mammalian subjects, said method comprising injecting the hydrogel formulation into the mammalian body, followed by release of the one or more pharmaceutically active ingredients from the hydrogel formulation, preferably wherein the one or more pharmaceutically active ingredients are selected from the group consisting of anti-tumor agents, chemotherapeutic agents, local anesthetics and combinations thereof.

## Patentansprüche

1. Supramolekulares Polymer, umfassend Polymerketten nach Formel (I): 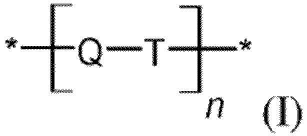 wobei:
• das durchschnittliche n in dem supramolekularen Polymer zwischen 2 und 16 liegt, einer der Bausteine Q über die mit einem Sternchen markierte Bindung mit einer Endgruppe verbunden ist und einer der Bausteine T über die mit einem Sternchen markierte Bindung mit einer Endgruppe verbunden ist;
• Baustein *-Q-* steht für: 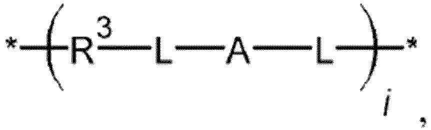 wobei das durchschnittliche *i* in dem supramolekularen Polymer zwischen 1,5 und 6,0 liegt;
• Baustein *-T-* steht für: 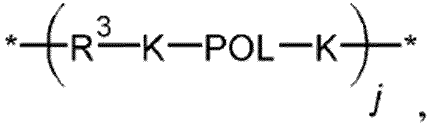 wobei das durchschnittliche *j* in dem supramolekularen Polymer zwischen 1 und 6 liegt;
• das supramolekulare Polymer ein durchschnittliches Molekulargewicht Mₙ von etwa 15 kDa bis etwa 150 kDa aufweist, wie mit Größenausschlusschromatographie (SEC) mit einem GPC-System unter Verwendung von RI-Detektion mit DMF bestimmt, umfassend 10 mM LiBr, bei 50 °C als Eluent, wobei die SEC-Daten relativ zu PEO/PEG-Standards sind;
• POL eine lineare hydrophile polymere Gruppe ist, die ein durchschnittliches Molekulargewicht Mₙ von etwa 1 kDa bis etwa 30 kDa aufweist;
• Rest A für Reste steht, die aus der Gruppe ausgewählt sind, bestehend aus den Formeln (II-A) bis (II-F), Tautomeren davon und Kombinationen davon, wobei A mit L über die mit einem Sternchen markierten Bindungen in den Formeln (II-A) bis (II-F) verbunden ist: 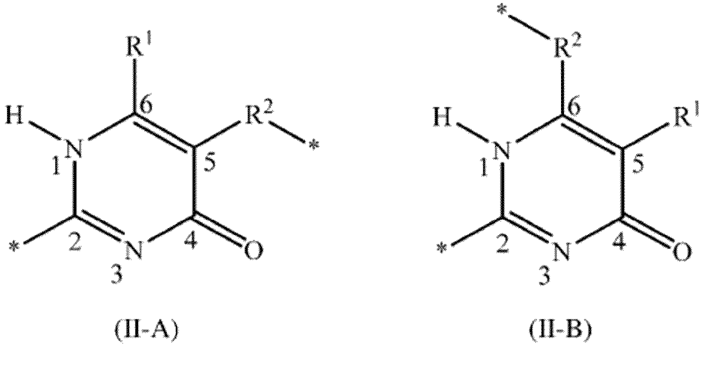 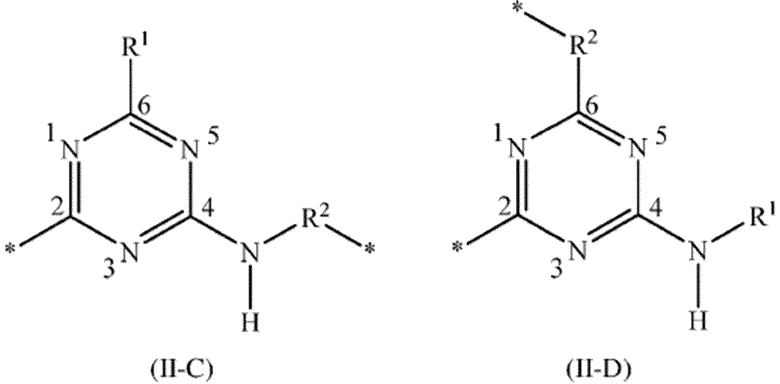 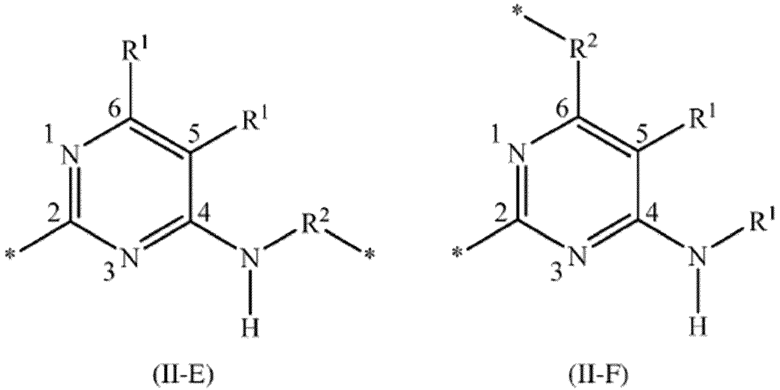
• jedes K eine Urethan-Verknüpfungsgruppe ist;
• jedes L unabhängig eine Urethan- oder Harnstoff-Verknüpfungsgruppe ist, mit der Maßgabe, dass ein beliebiger Rest A gemäß Formel (II-A) und (II-B) immer an eine Harnstoff-Verknüpfungsgruppe L an der 2-Position des 4-Pyrimidons gekoppelt ist, ein beliebiger Rest A gemäß Formel (II-C) und (II-D) immer an eine Harnstoff-Verknüpfungsgruppe L an der 2-Position des Triazins gekoppelt ist, und ein beliebiger Rest A gemäß Formel (II-E) und (II-F) immer an eine Harnstoff-Verknüpfungsgruppe L an der 2-Position des Pyrimidins gekoppelt ist;
• jedes R¹ unabhängig aus der Gruppe ausgewählt ist, bestehend aus Wasserstoff und C₁-C₂₀-Alkyl;
• R² aus der Gruppe ausgewählt ist, bestehend aus C₁-C₂₀-Alkylen, das optional mit O oder S substituiert ist; und
• R³ aus der Gruppe ausgewählt ist, bestehend aus linearen oder verzweigten C₂-C₂₀-Alkylengruppen und cyclischen C₃-C₂₄-Alkylengruppen.

2. Supramolekulares Polymer nach Anspruch 1, wobei A in Formel (I) für die folgenden Reste steht, die aus der Gruppe ausgewählt sind, bestehend aus Formel (II-A): 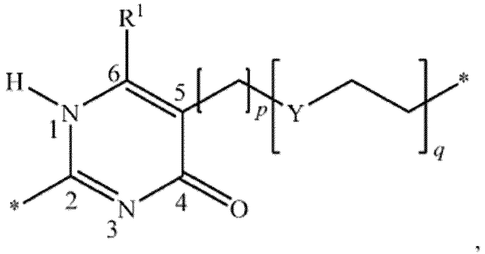 wobei:
R¹ aus der Gruppe ausgewählt ist, bestehend aus Wasserstoff und C₁-C₂₀-Alkyl;
Y O oder S ist;
*p* eine ganze Zahl von 1 bis 20 ist; und
*q* eine ganze Zahl von 0 bis 8 ist,
vorzugsweise wobei R¹ Methyl ist und:
a) *p* 2 ist und *q* 0 ist;
b) *p* 2 ist, *q* 1 ist und Y O ist; oder
c) *p* 4 bis 11 ist und *q* 0 ist.

3. Supramolekulares Polymer nach Anspruch 1 oder 2, wobei:
• ein beliebiger Rest A gemäß Formel (II-A) und (II-B) an eine Harnstoff-Verknüpfungsgruppe L an der 2-Position des 4-Pyrimidons und an eine Urethan-Harnstoff-Verknüpfungsgruppe L an der 5-Position des 4-Pyrimidons gekoppelt ist;
• ein beliebiger Rest A gemäß Formel (II-C) und (II-D) an eine Harnstoff-Verknüpfungsgruppe L an der 2-Position des Triazins und an eine Urethan-Verknüpfungsgruppe L an der 4-Position des Triazins gekoppelt ist,
• ein beliebiger Rest A gemäß Formel (II-E) und (II-F) an eine Harnstoff-Verknüpfungsgruppe L an der 2-Position des Pyrimidins und an eine Urethan-Verknüpfungsgruppe L an der 4-Position des Pyrimidins gekoppelt ist.

4. Supramolekulares Polymer nach einem der Ansprüche 1 bis 3, wobei R³ aus Hexylen ausgewählt ist,

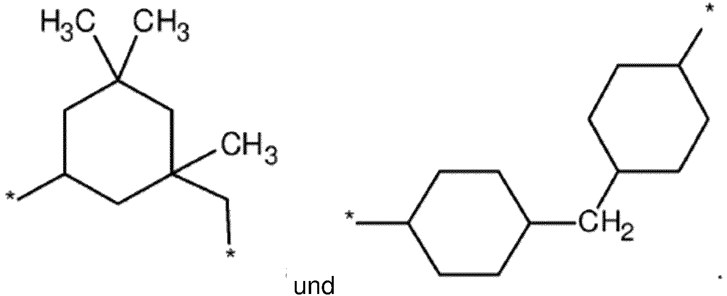

5. Supramolekulares Polymer nach einem der Ansprüche 1 bis 4, wobei POL ein lineares Polyethylenglykol ist, das ein durchschnittliches Molekulargewicht Mₙ zwischen 10 kDa und 25 kDa aufweist.

6. Prozess für die Herstellung eines supramolekularen Polymers durch Reagieren, optional in einem nicht reaktiven Lösungsmittel, einer Verbindung A', die aus der Gruppe ausgewählt ist, bestehend aus den Formeln (III-A) bis (III-F), Tautomeren davon und Kombinationen davon, mit einer Diisocyanatverbindung C' gemäß der Formel O=C=N-R³-N=C=O und einem Polymer HO-POL-OH: 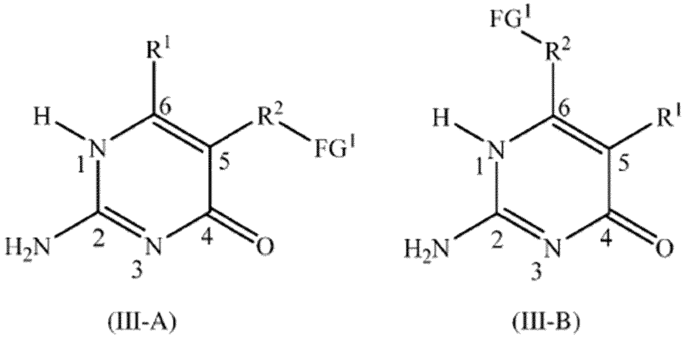 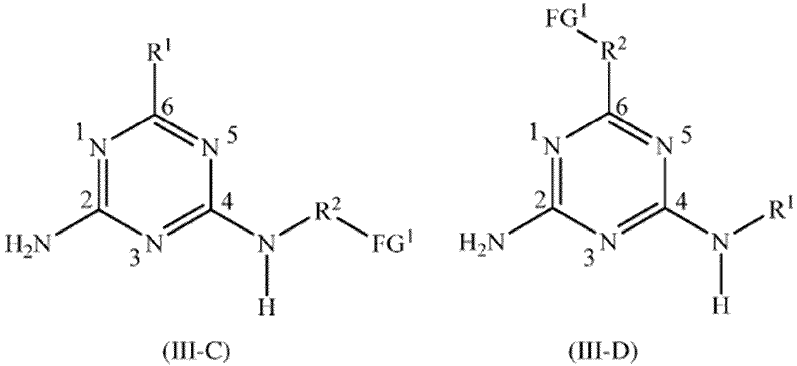 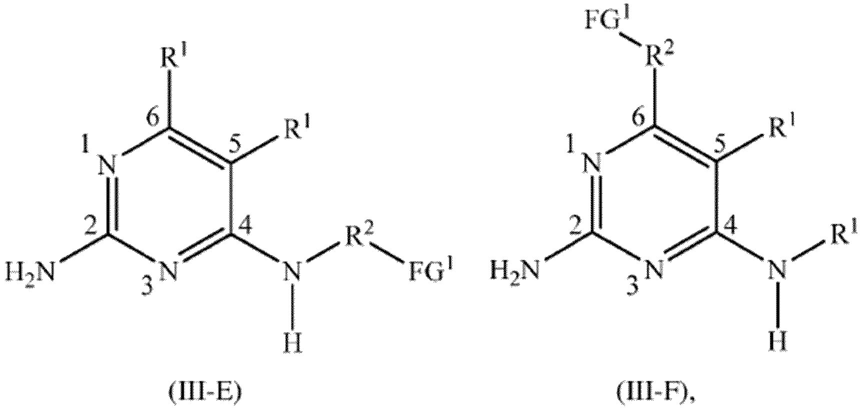
wobei R¹, R², R³ und POL wie in einem der Ansprüche 1 bis 6 definiert sind,
wobei FG¹ für eine funktionelle Gruppe steht, die aus OH und NH₂ ausgewählt ist, vorzugsweise OH,
wobei das während der Reaktion eingesetzte Molverhältnis von Verbindung A' zu HO-POL-OH zwischen 1,5 : 1,0 und 6,0 : 1,0 liegt, und wobei das Molverhältnis von Verbindung C' zu der während der Reaktion eingesetzten Summe von Verbindung A' und HO-POL-OH zwischen 1,1 : 1,0 und 0,9 : 1,0 liegt.

7. Prozess nach Anspruch 6, wobei A' für Reste steht, die aus der Gruppe ausgewählt sind, bestehend aus Formel (III-A): 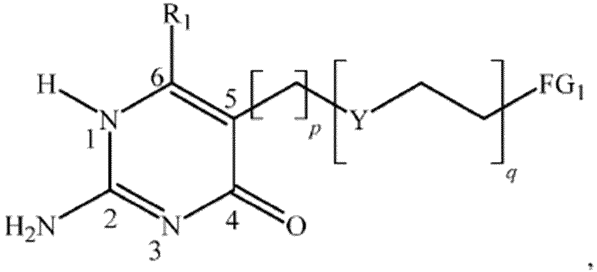 wobei:
R¹ aus der Gruppe ausgewählt ist, bestehend aus Wasserstoff und C₁-C₂₀-Alkyl;
Y O oder S ist;
*p* eine ganze Zahl von 1 bis 20 ist; und
*q* eine ganze Zahl von 0 bis 8 ist,
vorzugsweise wobei R¹ Methyl ist und:
a) *p* 2 ist, *q* 0 ist und FG¹ OH ist;
b) *p* 2 ist, *q* 1 ist, Y O ist und FG¹ OH ist;
c) *p* 4 bis 11 ist, *q* 0 ist und FG¹ OH ist; oder
d) *p* 4 bis 11 ist, *q* 0 ist und FG¹ NH₂ ist.

8. Prozess nach Anspruch 6 oder 7, wobei das Molverhältnis von Verbindung A' zu HO-POL-OH zwischen 1,5 : 1,0 und 4,0 : 1,0 liegt, und/oder wobei das produzierte supramolekulare Polymer ein supramolekulares Polymer gemäß Formel (I) wie in einem der Ansprüche 1 bis 5 definiert ist.

9. Supramolekulares Polymer, das durch den Prozess nach Anspruch 7 oder 8 erhältlich ist.

10. Hydrogelformulierung, umfassend zu 50,0-99,7 Gew.-% Wasser, zu 0,3-50,0 Gew.-% das supramolekulare Polymer nach einem der Ansprüche 1 bis 5 oder 9 und zu 0-30 Gew.-% weitere Bestandteile, bezogen auf das Gewicht der Hydrogelformulierung, wobei die Mengen an Wasser, supramolekularem Polymer und weiteren Bestandteilen zusammen 100 Gew.-% der Hydrogelformulierung ergeben.

11. Hydrogelformulierung nach Anspruch 10, die zu 0,9-10,0 Gew.-% das supramolekulare Polymer nach einem der Ansprüche 1 bis 5 oder 9, bezogen auf das Gewicht der Hydrogelformulierung, umfasst.

12. Hydrogelformulierung nach Anspruch 10 oder 11, die einen pH-Wert zwischen 8,5 und 14,0 aufweist und bei einer Temperatur zwischen 20 und 40 °C eine Flüssigkeit ist, vorzugsweise einen pH-Wert zwischen 8,5 und 14,0 aufweist und eine dynamische Viskosität bei 37 °C zwischen 0,01 und 20 Pa•s aufweist, gemessen mit einem Rheometer mit einer Platte-Platte-Geometrie bei einer Scherrate von 1 s⁻¹ und mit einem Spaltabstand von 0,50 mm.

13. Hydrogelformulierung nach Anspruch 10 oder 11, die bei einem pH-Wert zwischen 2,0 und weniger als 8,0 aufweist:
(i) Speichermodule *G*' von mindestens 20 Pa, vorzugsweise mindestens 200 Pa, am meisten bevorzugt mindestens 2000 Pa, über einen Frequenzbereich von 0,2 bis 20 Hz; und/oder
(ii) tan(*δ*)-Werte von weniger als 0,2, vorzugsweise zwischen 0,05 und 0,15, über einen Frequenzbereich von 0,2 bis 20 Hz,
wobei die Speichermodule *G*' und die tan(*δ*)-Werte mit einem Rheometer mit einer Platte-Platte-Geometrie und einem Spaltabstand von 0,50 mm bei Oszillationsfrequenzen zwischen 0,2 und 20 Hz und bei einer Temperatur von 37 °C gemessen werden.

14. Hydrogelformulierung nach einem der Ansprüche 10 bis 13, die zu 0,0001 bis 30 Gew.-% einen oder mehrere pharmazeutisch wirksame Bestandteile, bezogen auf das Gewicht der Hydrogelformulierung, umfasst.

15. Hydrogelformulierung nach Anspruch 12, umfassend zu 0,0001 bis 30 Gew.-% einen oder mehrere pharmazeutisch wirksame Bestandteile, bezogen auf das Gewicht der Hydrogelformulierung, zur Verwendung bei der Behandlung von onkologischen Erkrankungen, Herz-Kreislauf-Erkrankungen, orthopädischen Erkrankungen, Magen-Darm-Erkrankungen oder der Wundversorgung in Säugetiersubjekten, die Behandlung umfassend ein Injizieren der Hydrogelformulierung in den Säugetierkörper, gefolgt von einer Freisetzung des einen oder der mehreren pharmazeutisch wirksamen Bestandteile aus der Hydrogelformulierung, vorzugsweise wobei der eine oder die mehreren pharmazeutisch wirksamen Bestandteile aus der Gruppe ausgewählt sind, bestehend aus Antitumormitteln, chemotherapeutischen Mitteln, Lokalanästhetika und Kombinationen davon.

16. Hydrogelformulierung nach Anspruch 12, umfassend zu 0,0001 bis 30 Gew.-% einen oder mehrere pharmazeutisch wirksame Bestandteile, bezogen auf das Gewicht der Hydrogelformulierung, zur Verwendung in einem Verfahren für eine Prävention von Gewebeadhäsion oder in rekonstruktiver Chirurgie oder kosmetischer Chirurgie bei Säugetiersubjekten, das Verfahren umfassend das Injizieren der Hydrogelformulierung in den Säugetierkörper, gefolgt von der Freisetzung des einen oder der mehreren pharmazeutisch wirksamen Bestandteile aus der Hydrogelformulierung, vorzugsweise wobei der eine oder die mehreren pharmazeutisch wirksamen Bestandteile aus der Gruppe ausgewählt sind, bestehend aus Antitumormitteln, chemotherapeutischen Mitteln, Lokalanästhetika und Kombinationen davon.

## Revendications

1. Polymère supramoléculaire comprenant des chaînes polymères selon la Formule (I) : 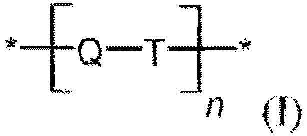 dans lequel :
• le *n* moyen dans le polymère supramoléculaire est compris entre 2 et 16, l'un des blocs de construction Q est relié à un groupe terminal par l'intermédiaire de la liaison marquée d'un astérisque, et l'un des blocs de construction T est relié à un groupe terminal par l'intermédiaire de la liaison marquée d'un astérisque ;
• le bloc de construction *-Q-* représente : 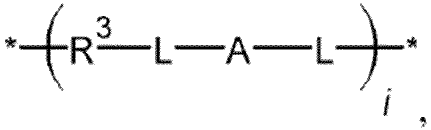 dans lequel le i moyen dans le polymère supramoléculaire est compris entre 1,5 et 6,0 ;
• le bloc de construction *-T-* représente : 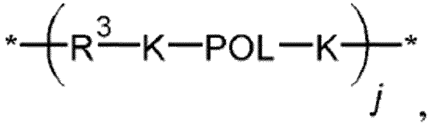 dans lequel le j moyen dans le polymère supramoléculaire est compris entre 1 et 6 ;
• le polymère supramoléculaire a une masse moléculaire moyenne Mₙ d'environ 15 kDa à environ 150 kDa, telle que déterminée par chromatographie d'exclusion stérique (SEC) avec un système GPC utilisant une détection RI avec du DMF comprenant 10 mM de LiBr à 50 °C en tant qu'éluant, les données de SEC étant relatives à des étalons PEO/PEG ;
• POL est un groupe polymère hydrophile linéaire ayant une masse moléculaire moyenne Mₙ d'environ 1 kDa à environ 30 kDa ;
• le fragment A représente des fragments choisis dans le groupe constitué de Formules (II-A) à (II-F), tautomères de ceux-ci et combinaisons de ceux-ci, dans lequel A est relié à L par l'intermédiaire des liaisons marquées d'un astérisque dans les Formules (II-A) à (II-F) : 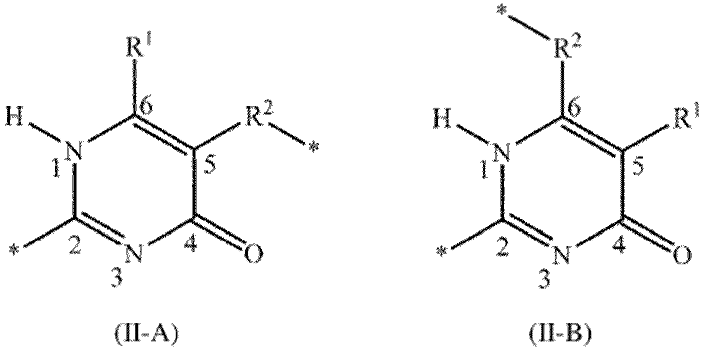 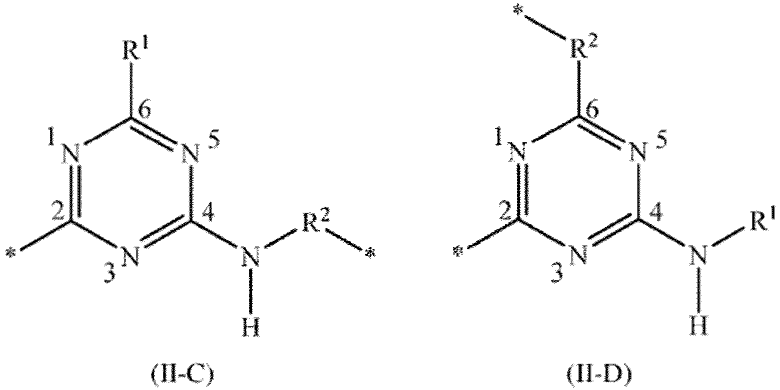 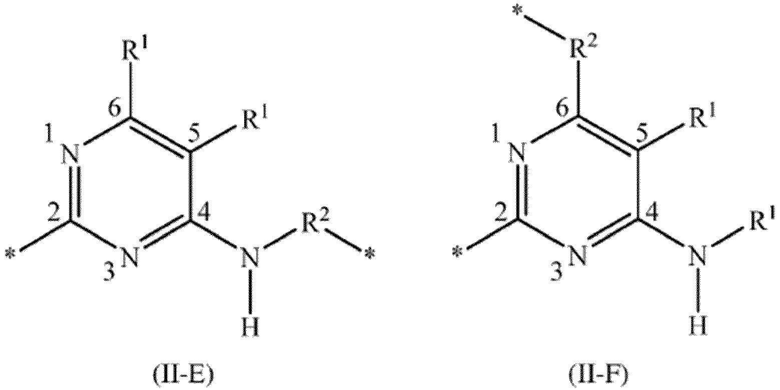
• chaque K est un groupe de liaison uréthane ;
• chaque L est indépendamment un groupe de liaison uréthane ou urée, à condition que tout fragment A selon la Formule (II-A) et (II-B) soit toujours couplé à un groupe de liaison urée L en position 2 de la 4-pyrimidone, que tout fragment A selon la Formule (II-C) et (II-D) soit toujours couplé à un groupe de liaison urée L en position 2 de la triazine, et que tout fragment A selon la Formule (II-E) et (II-F) soit toujours couplé à un groupe de liaison urée L en position 2 de la pyrimidine ;
• chaque R¹ est choisi indépendamment dans le groupe constitué d'hydrogène et alkyle en C₁-C₂₀ ;
• R² est choisi dans le groupe constitué d'alkylène en C₁-C₂₀, facultativement substitué par O ou S ; et
• R³ est choisi dans le groupe constitué de groupes alkylène linéaire ou ramifié en C₂-C₂₀ et de groupes alkylène cyclique en C₃-C₂₄.

2. Polymère supramoléculaire selon la revendication 1, dans lequel A dans la formule (I) représente les fragments suivants choisis dans le groupe constitué de Formule (II-A) : 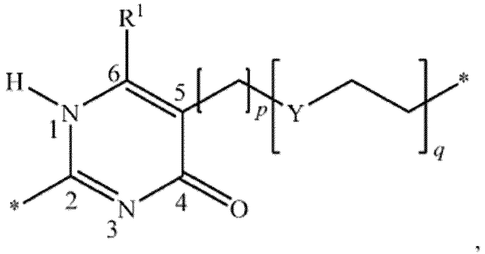 dans lequel :
R¹ est choisi dans le groupe constitué d'hydrogène et alkyle en C₁-C₂₀ ;
Y est O ou S ;
*p* est un entier allant de 1 à 20 ; et
*q* est un entier allant de 0 à 8,
de préférence dans lequel R¹ est un méthyle et :
a) *p* vaut 2 et *q* vaut 0 ;
b) *p* vaut 2, *q* vaut 1 et Y est O ; ou
c) *p* vaut de 4 à 11 et *q* vaut 0.

3. Polymère supramoléculaire selon la revendication 1 ou 2, dans lequel :
• tout fragment A selon la Formule (II-A) et (II-B) est couplé à un groupe de liaison urée L en position 2 de la 4-pyrimidone et à un groupe de liaison uréthane urée L en position 5 de la 4-pyrimidone ;
• tout fragment A selon la Formule (II-C) et (II-D) est couplé à un groupe de liaison urée L en position 2 de la triazine et à un groupe de liaison uréthane L en position 4 de la triazine,
• tout fragment A selon la Formule (II-E) et (II-F) est couplé à un groupe de liaison urée L en position 2 de la pyrimidine et à un groupe de liaison uréthane L en position 4 de la pyrimidine.

4. Polymère supramoléculaire selon l'une quelconque des revendications 1 à 3, dans lequel R³ est choisi parmi hexylène,

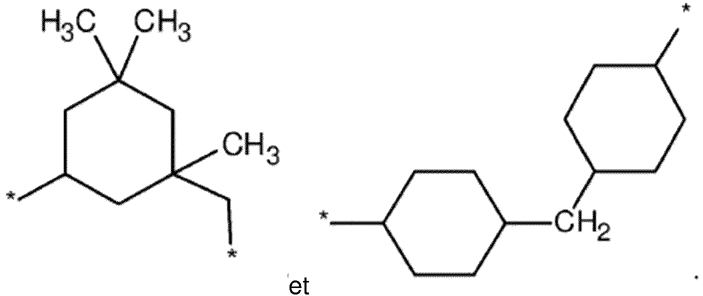

5. Polymère supramoléculaire selon l'une quelconque des revendications 1 à 4, dans lequel POL est un polyéthylène glycol linéaire ayant une masse moléculaire moyenne Mₙ comprise entre 10 kDa et 25 kDa.

6. Procédé de fabrication d'un polymère supramoléculaire par réaction, facultativement dans un solvant non réactif, d'un composé A' choisi dans le groupe constitué de Formules (III-A) à (III-F), tautomères de ceux-ci et combinaisons de ceux-ci avec un composé diisocyanate C' selon la Formule O=C=N-R³-N=C=O et un polymère HO-POL-OH : 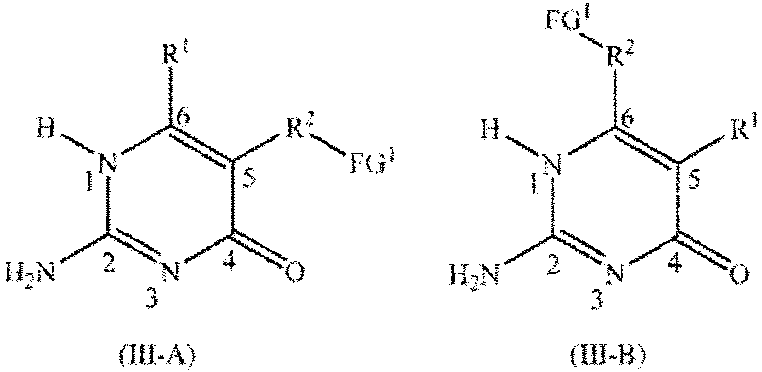 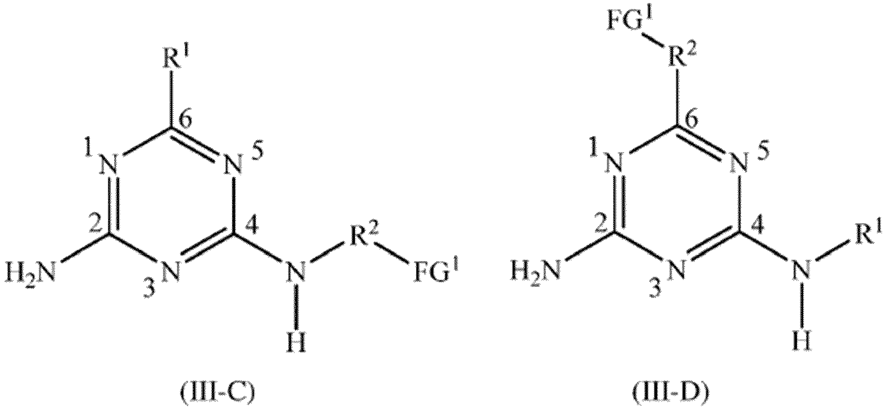 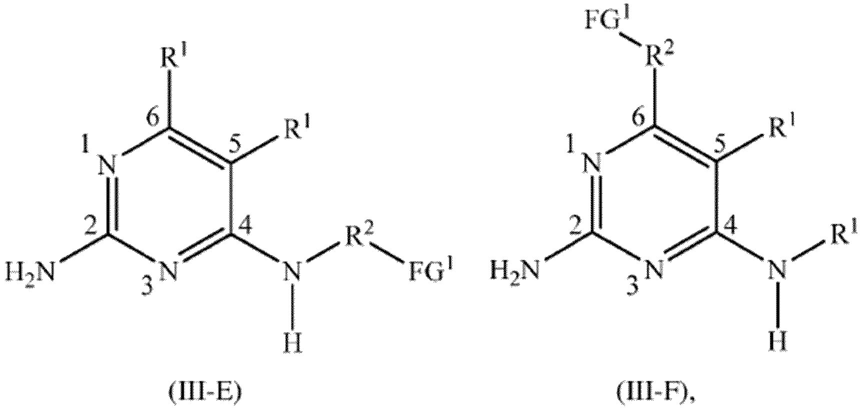
dans lequel R¹, R², R³ et POL sont tels que définis dans l'une quelconque des revendications 1 à 6,
dans lequel FG¹ représente un groupe fonctionnel choisi parmi OH et NH₂, de préférence OH,
dans lequel le rapport molaire du composé A' au HO-POL-OH appliqué pendant la réaction est compris entre 1,5:1,0 et 6,0:1,0, et dans lequel le rapport molaire du composé C' à la somme du composé A' et de HO-POL-OH appliqués pendant la réaction est compris entre 1,1:1,0 et 0,9:1,0.

7. Procédé selon la revendication 6, dans lequel A' représente des fragments choisis dans le groupe constitué de Formule (III-A) : 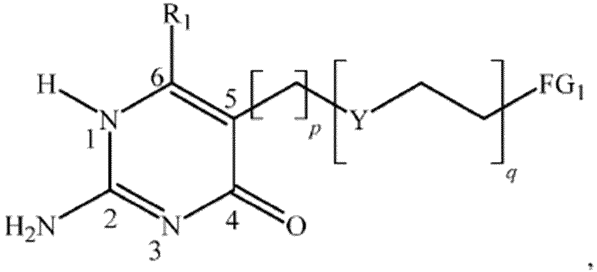 dans lequel :
R¹ est choisi dans le groupe constitué d'hydrogène et alkyle en C₁-C₂₀ ;
Y est O ou S ;
*p* est un entier allant de 1 à 20 ; et
*q* est un entier allant de 0 à 8,
de préférence dans lequel R¹ est un méthyle et :
a) *p* vaut 2, *q* vaut 0 et FG¹ est OH ;
b) *p* vaut 2*, q* vaut 1, Y est O et FG¹ est OH ;
c) *p* vaut de 4 à 11, *q* vaut 0 et FG¹ est OH ; ou
d) *p* vaut de 4 à 11, *q* vaut 0 et FG¹ est NH₂.

8. Procédé selon la revendication 6 ou 7, dans lequel le rapport molaire du composé A' au HO-POL-OH est compris entre 1,5:1,0 et 4,0:1,0, et/ou dans lequel le polymère supramoléculaire produit est un polymère supramoléculaire selon la Formule (I) tel que défini dans l'une quelconque des revendications 1 à 5.

9. Polymère supramoléculaire pouvant être obtenu par le procédé selon la revendication 7 ou 8.

10. Formulation d'hydrogel comprenant 50,0 à 99,7 % en poids d'eau, 0,3 à 50,0 % en poids du polymère supramoléculaire selon l'une quelconque des revendications 1 à 5 ou 9, et 0 à 30 % en poids d'ingrédients supplémentaires, par rapport au poids de la formulation d'hydrogel, dans laquelle les quantités d'eau, de polymère supramoléculaire et d'ingrédients supplémentaires totalisent 100 % en poids de la formulation d'hydrogel.

11. Formulation d'hydrogel selon la revendication 10, qui comprend 0,9 à 10,0 % en poids du polymère supramoléculaire selon l'une quelconque des revendications 1 à 5 ou 9, par rapport au poids de la formulation d'hydrogel.

12. Formulation d'hydrogel selon la revendication 10 ou 11, qui a un pH compris entre 8,5 et 14,0 et est un liquide à une température comprise entre 20 et 40 °C, ayant de préférence un pH compris entre 8,5 et 14,0 et ayant une viscosité dynamique à 37 °C comprise entre 0,01 et 20 Pa•s, telle que mesurée avec un rhéomètre à géométrie plan-plan à un taux de cisaillement de 1 s⁻¹ et avec une distance d'entrefer de 0,50 mm.

13. Formulation d'hydrogel selon les revendications 10 ou 11, ayant, à un pH compris entre 2,0 et moins de 8,0 :
(i) des modules de conservation *G*' d'au moins 20 Pa, de préférence d'au moins 200 Pa, le plus préférablement d'au moins 2 000 Pa, sur une plage de fréquences de 0,2 à 20 Hz ; et/ou
(ii) des valeurs de tan(*δ*) inférieures à 0,2, de préférence comprises entre 0,05 et 0,15, sur une plage de fréquences de 0,2 à 20 Hz,
dans laquelle les modules de conservation *G*' et les valeurs de tan(*δ*) sont mesurés avec un rhéomètre à géométrie plan-plan et une distance d'entrefer de 0,50 mm, à des fréquences oscillatoires comprises entre 0,2 et 20 Hz et à une température de 37 °C.

14. Formulation d'hydrogel selon l'une quelconque des revendications 10 à 13, qui comprend 0,0001 à 30 % en poids d'un ou plusieurs principes pharmaceutiquement actifs, par rapport au poids de la formulation d'hydrogel.

15. Formulation d'hydrogel selon la revendication 12, comprenant 0,0001 à 30 % en poids d'un ou plusieurs principes pharmaceutiquement actifs, par rapport au poids de la formulation d'hydrogel, pour une utilisation dans le traitement de maladies oncologiques, maladies cardiovasculaires, maladies orthopédiques, maladies gastro-intestinales ou le soin des plaies chez des sujets mammifères, ledit traitement comprenant l'injection de la formulation d'hydrogel dans le corps du mammifère, en faisant suivre par la libération du ou des principes pharmaceutiquement actifs à partir de la formulation d'hydrogel, de préférence dans laquelle le ou les principes pharmaceutiquement actifs sont choisis dans le groupe constitué d'agents antitumoraux, agents chimiothérapeutiques, anesthésiques locaux et combinaisons de ceux-ci.

16. Formulation d'hydrogel selon la revendication 12, comprenant 0,0001 à 30 % en poids d'un ou plusieurs principes pharmaceutiquement actifs, par rapport au poids de la formulation d'hydrogel, pour une utilisation dans un procédé de prévention de l'adhérence tissulaire ou en chirurgie reconstructrice ou en chirurgie esthétique chez des sujets mammifères, ledit procédé comprenant l'injection de la formulation d'hydrogel dans le corps du mammifère, en faisant suivre par la libération du ou des principes pharmaceutiquement actifs à partir de la formulation d'hydrogel, de préférence dans laquelle le ou les principes pharmaceutiquement actifs sont choisis dans le groupe constitué d'agents antitumoraux, agents chimiothérapeutiques, anesthésiques locaux et combinaisons de ceux-ci.
